# EUROPEAN PATENT SPECIFICATION

(11) **EP 2 567 236 B1**
(45) Date of publication and mention of the grant of the patent: **04.11.2015**
(21) Application number: 11719459.7
(22) Date of filing: 03.05.2011
(51) Int. Cl.: G01N 33/68

(54) **Test for male fertility using vitamin D metabolising pathways**
Test der männlichen Fertilität
Test pour la fertilité masculine

(30) Priority: 03.05.2010 EP 10161711
(43) Date of publication of application: 13.03.2013
(73) Proprietor: Rigshospitalet, Copenhagen University Hospital, 2100 Copenhagen Ø (DK)
(72) Inventor: JENSEN, Martin, Blomberg, DK-2100 Copenhagen Ø (DK); NIELSEN, John, Erik, DK-2300 Copenhagen S (DK); JØRGENSEN, Niels, DK-2300 Copenhagen S (DK); ALMSTRUP, Kristian, DK-4000 Roskilde (DK); LEFFERS, Henrik, DK-2200 Copenhagen N (DK)
(74) Representative: Plougmann & Vingtoft A/S
(86) International application number: PCT/DK2011/050150
(87) International publication number: WO 2011/137906

(56) References cited:
- CORBETT S T ET AL: "Vitamin D receptor found in human sperm", UROLOGY, BELLE MEAD, NJ, US LNKD- DOI:10.1016/J.UROLOGY.2006.09.011, vol. 68, no. 6, 1 December 2006 (2006-12-01), pages 1345-1349, XP025075810, ISSN: 0090-4295 [retrieved on 2006-12-01]
- AQUILA SAVERIA ET AL: "Human male gamete endocrinology: 1alpha, 25-dihydroxyvitamin D3 (1,25(OH)2D3) regulates different aspects of human sperm biology and metabolism", REPRODUCTIVE BIOLOGY AND ENDOCRINOLOGY, XX, XX, vol. 7, no. 1, 30 November 2009 (2009-11-30), page 140, XP021068191, ISSN: 1477-7827 cited in the application
- BLOMBERG JENSEN MARTIN ET AL: "Vitamin D receptor and vitamin D metabolizing enzymes are expressed in the human male reproductive tract.", HUMAN REPRODUCTION (OXFORD, ENGLAND) MAY 2010 LNKD- PUBMED:20172873, vol. 25, no. 5, 18 February 2010 (2010-02-18), pages 1303-1311, XP002589754, ISSN: 1460-2350 cited in the application
- KINUTA KEIKO ET AL: "Vitamin D is an important factor in estrogen biosynthesis of both female and male gonads", ENDOCRINOLOGY, vol. 141, no. 4, April 2000 (2000-04), pages 1317-1324, XP002589755, ISSN: 0013-7227 cited in the application

## Description

### Field of the invention

The present invention relates generally to a method for predicting the fertility potential in a male mammal. In particular it relates to a method for predicting the fertility potential in a male mammal by determining the expression of CYP24A1 in a semen sample. The expression level of CYP24A1 is then indicative of the fertility potential of the subject.

An object of the present invention relates to a method, which can predict whether the semen quality is sufficient to achieve pregnancy spontaneously or if the couple should proceed to assisted reproduction or further investigation.

### Background of the invention

### Fertility

Decreased semen quality is a major factor of male infertility. Semen quality is a measure of the ability of the semen to accomplish fertilization. Evaluation of male fertility potential is today basically conducted through semen analysis. A semen analysis evaluates certain characteristics of a male's semen and the spermatozoa contained in the semen. The characteristics measured by the current tests for semen analysis are only some of the clinical important factors for semen quality. The most common variables measured to evaluate sperm quality are: sperm count, motility and morphology. Other variables are volume, fructose level and pH.

In general, if a person has a high sperm number (above 40 millions), above 50% motile sperm and more than 12 % morphological normal spermatozoa the person is regarded to have a normal fertility potential. This is not an accurate estimate of male fertility potential and the result of the semen analysis does not always correlate with the ability to have children. This is also illustrated by the fact that WHO recently has changed the reference levels as a large proportion of the male population otherwise would be regarded as having a fertility potential below normal. No precise method for predicting the male fertility potential exists presently.

More than 9 % of all babies are born after assisted reproductive methods in Denmark, and the incidence of infertility is increasing worldwide. About 50% of cases are attributable to the male partner and the first step in the clinical evaluation involves semen analysis to evaluate male fertility potential. The clinicians use the analysis in deciding the appropriate reproductive method.

It is estimated that some 1.400.000 semen analyses are performed each year worldwide.

Semen analysis is currently a cumbersome and lengthy process. It is performed by standard microscopy and is based on the subjective analysis of three key parameters: motility, morphology, and total sperm number. The analysis has several drawbacks as it necessitates a trained medical staff, presence of the patient with a fresh semen sample (less than 1 hour old), laboratory facilities etc. making it a costly and time consuming procedure. Logistically, the analysis requires the patient to deliver a semen sample at the laboratory where the testing is performed, which for many patients is considered a major embarrassment. Furthermore, semen analyses using the current methods is highly prone to intra- and inter observer variability due to the subjective assessment of the key parameters, which severely can affect the utility of the analysis, whereby the clinician is troubled in the guidance of the couple to the relevant assisted reproductive method.

Due to above mentioned problems with the currently used methods, a large need exist for a new method for semen analysis, which accurately and objectively can assess male fertility potential in order to stratify patients into the correct reproductive method. The problems with the existing analysis are illustrated by the latest WHO recommendations from 2010, where the reference values for normal semen analysis were changed and caused a huge debate in fertility and andrology meetings worldwide.

In particular it would be of great benefit to have a test, which could predict whether the person should continue at home the natural way or with the mild insemination or if the couple should proceed to the more invasive IVF or ICSI. Thus, developing a marker that can guide the doctor in this choice would be a clear advantage.

### Vitamin D

Vitamin D is a key regulator of calcium homeostasis and needs to be activated by two enzymatic steps, before it binds with high affinity to the vitamin D receptor (VDR) and elicit its effects. Synthesis normally starts in the skin, where 7-hydrocholesterol is converted to vitamin D after UVB radiation. Vitamin D is subsequently metabolized by the hepatic 25-hydroxylases (CYP2R1 or CYP27A1), before 25-hydroxycholecalciferol (25(OH)D₃) relocates to the circulation, where the renal 1alpha-hydroxylase (CYP27B1) converts 25(OH)D₃ to the active 1,25(OH)₂D₃ (calcitriol). Calcitriol binds and activates VDR in target cells, until the 24-hydroxylase (CYP24A1) inactivates it (Prosser and Jones, 2004).

Some studies have suggested that vitamin D is important for male reproduction in mammals. This is most convincingly shown in rodents, where vitamin D deficiency caused reduced sperm counts and lower fertility rates in females inseminated with sperm from deficient males (Jensen et al., 2009).

A study by Aquila et al 2008 showed that the action of 1,25(OH)₂D₃ depends on the concentration as low concentrations seems to induce sperm survival whereas higher concentration seemed to be ineffective or did not shown an increased effect (Aquila et al., 2008).

Thus, the precise role of vitamin D in reproduction is still unclear, especially in humans.

### The vitamin D metabolizing machinery

Vitamin D acts through the VDR, and VDR expression has been shown in testis from rodents, chickens, roosters, humans and recently also in mature human spermatozoa. The importance of this expression is highlighted in VDR knockout mice, which show decreased sperm counts, reduced sperm motility and histological abnormalities of the testis, which unlike the vitamin D deficient male rats only partly can be restored by calcium supplements (Kinuta et al., 2000).

Another study by Aquila et al. 2009 showed that human spermatozoa express VDR and that sperm contain 1,25(OH)₂D₃ and furthermore that 1,25(OH)₂D₃ through VDR increases intracellular Ca²⁺ levels, motility and acrosin activity (Aquila et al., 2009).

The liver and kidney were previously thought to be the only organs with the ability to activate vitamin D, but extra-renal expression of CYP27B1, CYP24A1 and CYP27A1 have been shown in tissues thought only to be vitamin D responsive (Nagakura et al., 1986).

Thus the cellular response to vitamin D is complex, since it is not solely dependent on VDR expression, but also on cellular uptake of circulating vitamin D and presence and activity of vitamin D metabolizing enzymes.

The role of the vitamin metabolizing enzymes in reproduction is poorly studied, but Blomberg Jensen et al 2011 showed that activated VD increases intracellular calcium in human spermatozoa by releasing calcium concentration from an intracellular storage. This calcium release has important functional consequences as activated VD is able to increase sperm motility and induce the acrosome reaction needed for fertilization under capacitating conditions.

A study by Panda et al. 2000 suggested that the 1-hydroxylase (CYP27B1) plays a role in female reproduction by using a mouse model of CYP27B1 deficiency. However the reproductive organs of male CYP27B1 null mutant mice appeared grossly normal (Panda et al 2001).

Recently is was shown by Blomberg Jensen et al. 2010 that the VDR, activating (CYP2R1, CYP27B1 and CYP27A1) and inactivating (CYP24A1) enzymes are expressed in human germ cells, epithelium of the epididymis, prostate, seminal vesicle and in mature human spermatozoa, where VDR and the metabolising enzymes co-localise in the postacrosomal region, neck and midpiece. (Jensen et al., 2009)

Corbett S.T. et al describes that vitamin D receptors where reduced in patients with infertility and having reduced sperm levels. However, this document is silent in respect of infertility tests. (Vitmain D receptor found in human sperm, Urology, 2006, Dec; 68(6):1345-9).

It has however never been tested or suggested that it is possible to predict the male fertility potential by determining the expression of VDR and/or at least one of the vitamin D metabolising enzymes as in the present invention.

Thus it is here shown for the first time that determination of the expression of VDR, CYP24A1, CYP2R1, CYP27B1 and/or CYP27A1 can be used to predict the fertility potential in a male mammal and thus as a marker for sperm quality.

### Summary of the invention

The present invention provides a method for predicting the fertility potential of a male mammal.

One aspect of the invention relates to the use of the expression of CYP24A1 as a marker for male fertility potential. The expression of VDR and/or vitamin D metabolizing enzymes is shown to correlate with semen quality.

Thus, the present invention provides a method comprising the steps of providing a semen sample from said male mammal and determining the expression level of CYP24A1, selecting a desired sensitivity, selecting a desired specificity, determining whether said male mammal is likely to have a normal fertility potential, if said determined expression level is equal to or above a reference level and/or determining whether said mammal is unlikely to have a normal fertility potential, if said determined expression level is below the reference level.

The present inventors have demonstrated the principle of predicting the male fertility potential in humans using a method based on obtaining a semen sample from a male individual and subsequent determination of CYP24A1.

The inventors have shown that all these proteinsare novel markers for male fertility potential that can distinguish infertile men from fertile. The best marker seem to be expression of CYP24A1 that correlates significantly with all the traditional semen variables: total sperm number (p<0.0005), motility (p<0.002) and morphology (p<0.004), hence making CYP24A1 an ideal objective marker for male fertility potential.

Furthermore the inventors found that the expression of CYP24A1 at the annulus is so distinct that it is easy to recognize. Since it is so easy to recognize the detection of CYP24A1 can be done by a machine.

### Detailed description

The present invention concerns a novel method to predict the fertility potential in a male mammal.

One aspect of the invention relates to the use according to claim 9. The expression of CYP24A1 correlates with sperm quality parameters. Thus determining the expression of CYP24A1 can be used as a marker of male fertility.

The expression of at least one protein of the vitamin D metabolizing machinery can be detected using ligands such as antibodies specific for the proteins of the vitamin D metabolizing machinery and/or by measuring the level of expression of genes encoding the proteins of the vitamin D metabolizing machinery.

Thus, the present invention provides means for predicting male fertility potential i.e. whether a male has a normal fertility potential or a fertility potential below normal.

The present invention provides a simple method for predicting the fertility potential of a male mammal, which is more precise than the traditional method using semen analysis.

The male fertility potential can be determined by measuring the level of CYP24A1 in a semen sample. The level of the at least one protein of vitamin D metabolizing machinery may be detected using ligands such as antibodies specific for said protein of vitamin D metabolizing machinery.

A large proportion of ejaculated spermatozoa from fertile men had a concomitant expression of VDR and the VD metabolising enzymes in the ejaculated spermatozoa compared to few in spermatozoa from infertile men. The present inventors have demonstrated the principle of predicting the male fertility potential in humans using the expression of CYP24A1 as an example. This has now been extended by showing that VDR, CYP2R1, CYP27A1, CYP27B1 and CYP24A1 were expressed with comparable strength using western blot to detect the protein in samples of spermatozoa from each tested individual.

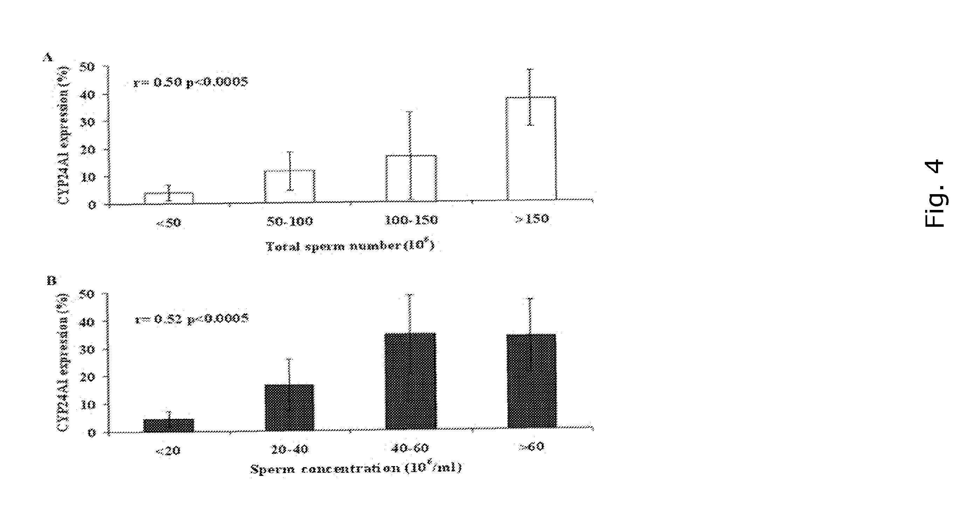

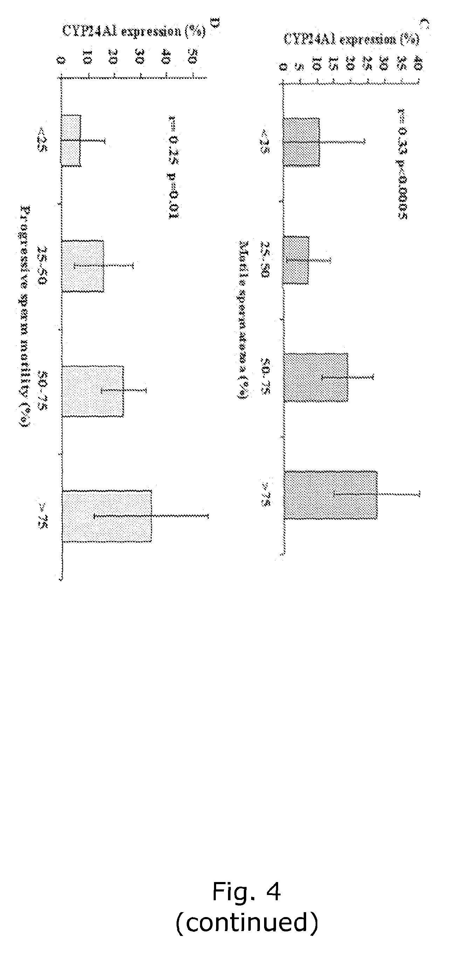

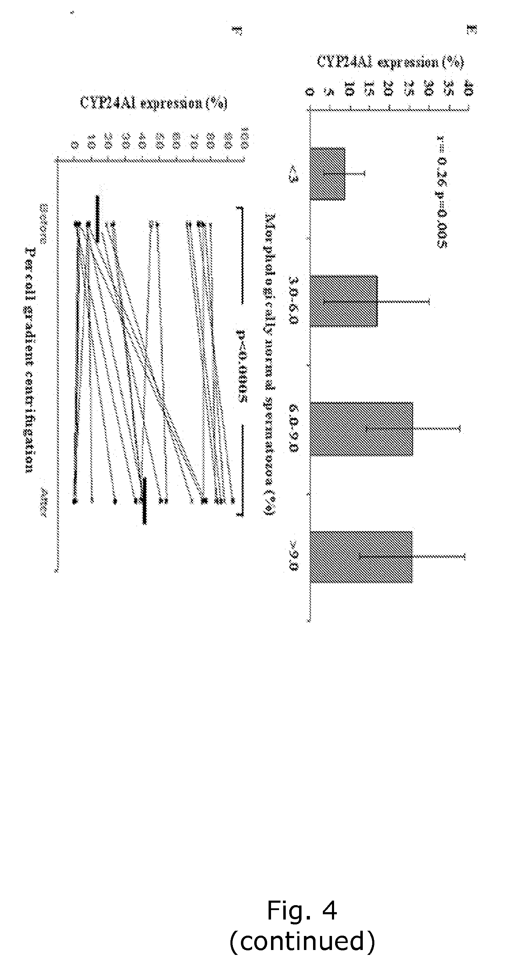

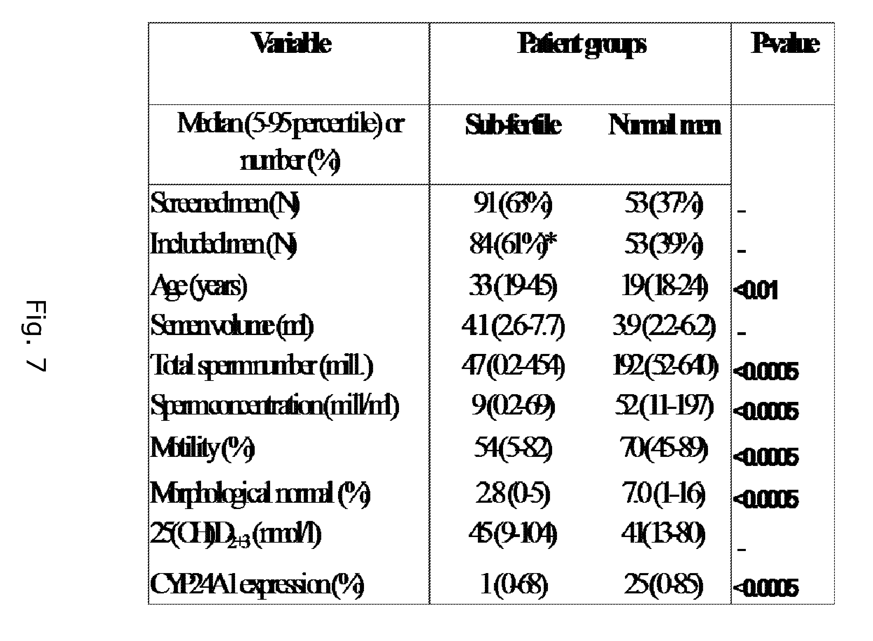

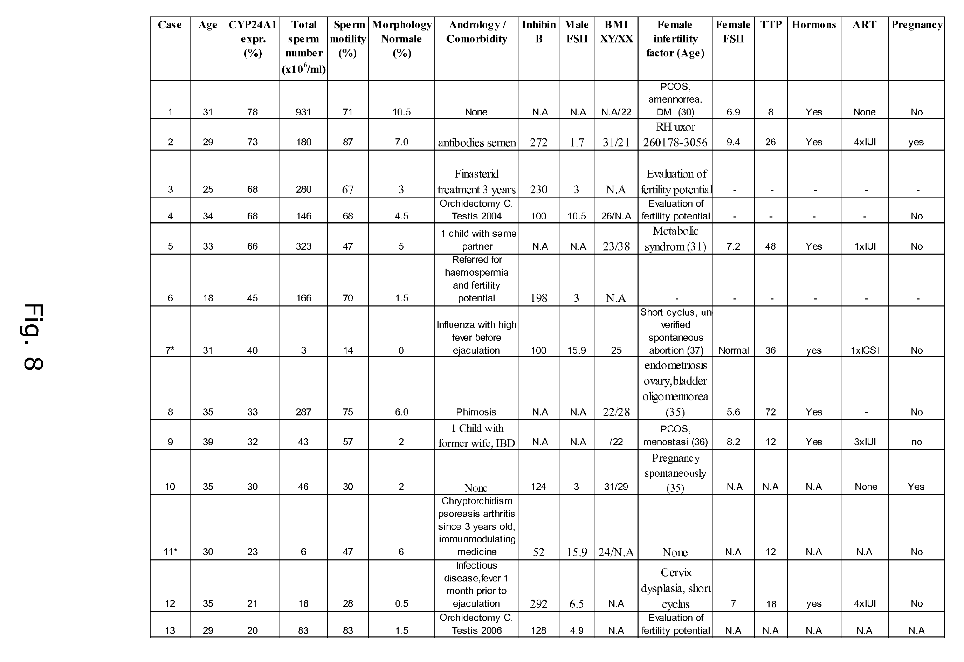

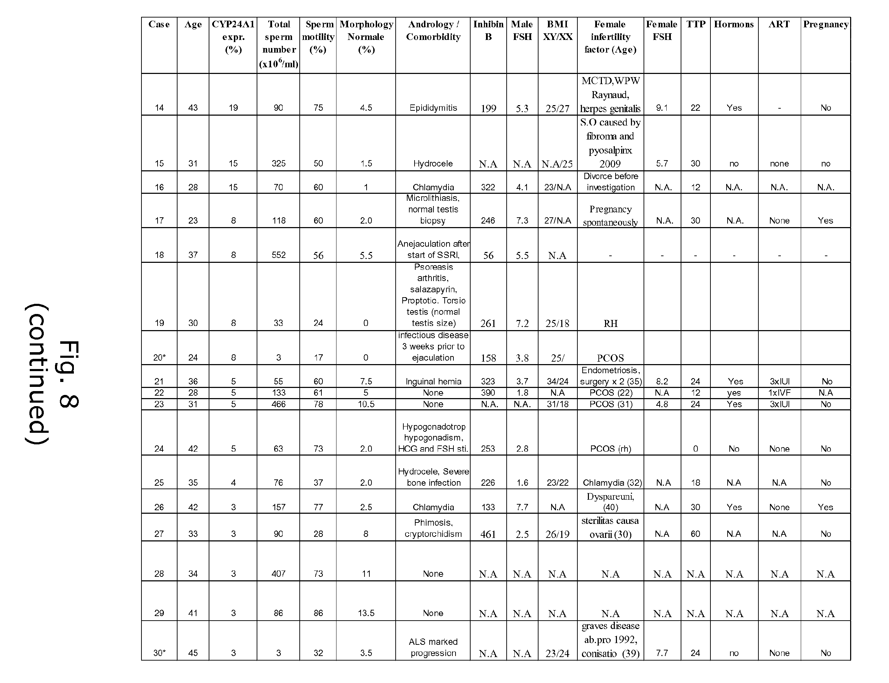

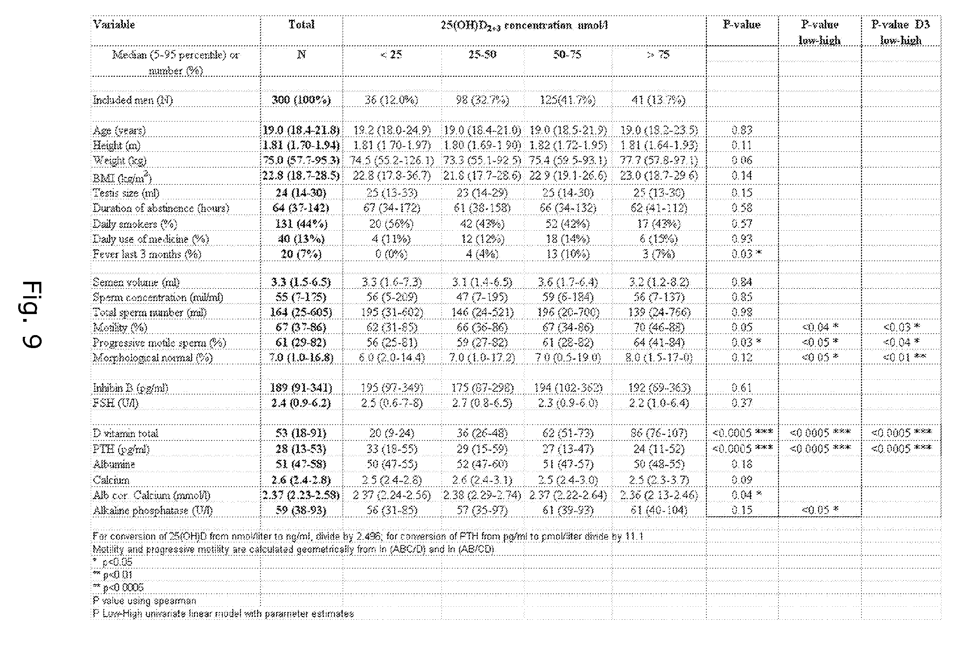

The inventors investigated 53 young military conscripts (control) and 91 men referred for semen analysis at an andrological clinic (subfertile) (figure 7). The inventors found a highly significant difference (p<0.0005) in the proportion of spermatozoa expressing CYP24A1 at the annulus between the two groups, although some of the subfertile men had a high proportion of spermatozoa expressing CYP24A1. Surprisingly were the subfertile men with CYP24A1 expression > 2% virtually normospermic (figure 8). They had a high total sperm number, >50% motile spermatozoa, high Inhibin B levels, low FSH, although a few men had a low total sperm number despite > 2% of spermatozoa were expressing CYP24A1 at the annulus. To determine whether these men where subfertile or not, the inventors evaluated the medical history, spouse history and fertility problems and the cause for referral to the clinic (figure 8). Only 1/30 (3%) of the subfertile men with >2% spermatozoa expressing CYP24A1 had solemnly a male fertility problem. Some of the men had low sperm counts and many immotile spermatozoa, but they could all be explained by reversible causes or co-morbidity and not a primary testis problem. These men were classified as normospermic subfertile, which increased the difference between the subfertile and control group (p<0.0005). Semen quality differs significantly between the subfertile and the control group, and CYP24A1 expression seems to be a positive predictor of male fertility potential, because absence of CYP24A1 expression was also seen in the military conscripts, despite some of them had a high sperm number and many motile sperm. To evaluate its role as a positive predictor of male fertility potential, the CYP24A1 expression was evaluated in relation to semen quality variables. The inventors found a significant association (p<0.005) to total sperm count, sperm motility, morphology and Inhibin B levels in (figure 4).

Thus the inventors found that e.g. CYP24A1 expression is significantly correlated with all semen quality parameters. This finding was further validated by performing a Percoll centrifugation of the sperm to retrieve the good quality sperm. Percoll centrifugation separates the progressive motile sperm with normal morphology from the remnants of the semen sample. The inventors found a significant higher expression of e.g. CYP24A1 after Percoll centrifugation which indicates that it is valid marker for good semen quality.

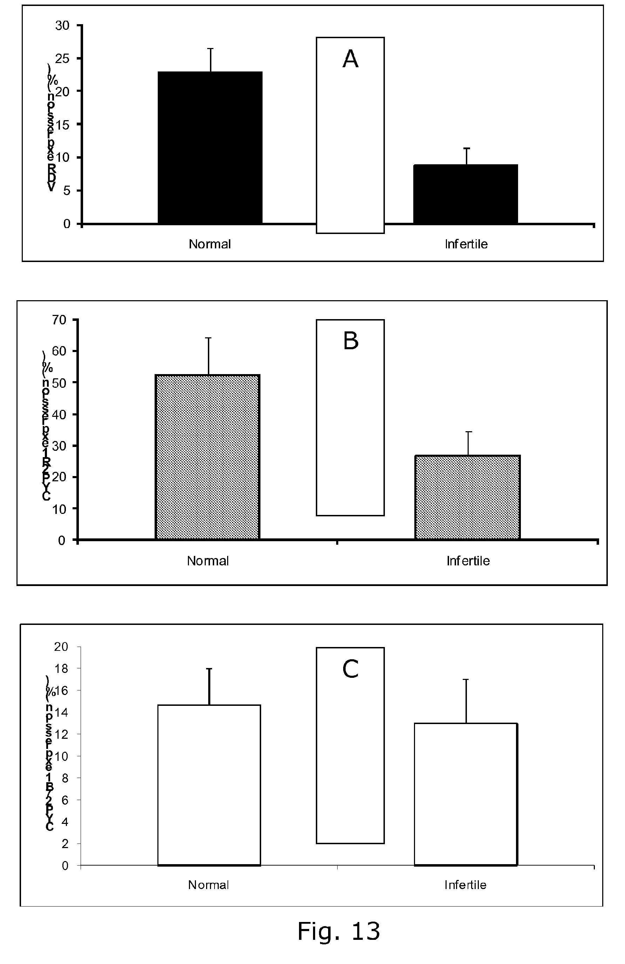

Expression of VDR, CYP2R1, CYP27B1 was also investigated in semen samples from normal and infertile men (Figure 13). All investigated proteins had a higher expression in spermatozoa from normal men compared with sperm from infertile men, and presence of these proteins can thus assist in assessing male fertility potential. To ensure that the same spermatozoa express both VDR and CYP24A1 we conducted double staining of semen samples from 3 different men.More than 80 % of the spermatozoa were either expressing both proteins or none of them indicating that high quality spermatozoa express either all the proteins in the vitamin D metabolizing machinery or none of them.

CYP2R1, CYP27A1, CYP27B1, VDR and CYP24A1 may thus serve as a marker for male fertility potential that can distinguish infertile men from fertile men and potentially predict which type of assisted reproductive techniques (ART) that is needed.

Thus the method can identify persons with a normal or reduced male fertility potential.

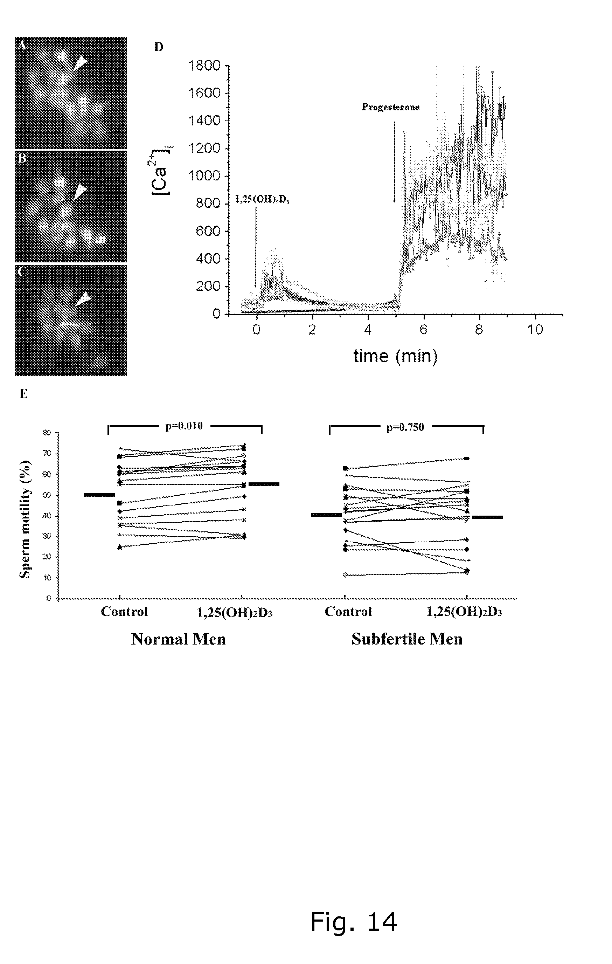

Since presence of the VD metabolizing enzymes is concomitant with the VDR may the expression of the marker have functional consequences; activated VD increases intracellular calcium through VDR activation in human spermatozoa from normal and since infertile men have fewer sperm expressing the metabolizing enzymes and the VDR, they are likely to be unresponsive. This suggestion was confirmed by showing that some spermatozoa was unresponsive to activated VD and spermatozoa from infertile men did not increase in sperm motility following treatment with activated VD unlike spermatozoa from normal men (Figure 14). These functional studies support that the expression of the VD metabolizing machinery differs between normal and infertile men and it has functional consequences.

This may have relevance for another aspect of the present invention, which relates to a method that can predict the male fertility potential. Based on the result determine whether the person should continue at home the natural way or with the mild insemination or if the couple should proceed to the more invasive IVF or ICSI. Thus, evaluation of the expression of the at least one protein of the vitamin D metabolising machine is a possible replacement or an attractive add-on to standard semen analysis to determine whether ART is needed and which ART method such as but not limited to intrauterine insemination (IUI), in vitro fertilisation (IVF) and/or intracytoplasmatic sperm injection (ICSI) that is most suitable for the particular patient.

Furthermore the inventors found that the expression of CYP24A1 at the annulus is so distinct that it is easy to recognize. Since it is so easy to recognize the detection of CYP24A1 can be done by a machine.

The method described in the present invention has several advantages over the current method:
▪ CYP24A1 or similar markers as described herein analysis can be quantified using automated methods thereby making the semen analysis objective, reliable and scalable for high-throughput.
▪ The test can be performed in less time and will not require trained medical staff to the same degree as the current test.
▪ The analysis can be performed on fixed semen samples enabling the patient to make the sample in the privacy of his own home. Hereafter the sample can be mailed to the hospital/clinic.
▪ It could potentially be possible to make a pregnancy-like test, so that the entire analysis can be performed by the patient at home (home testing).
▪ This new method is reliable with high sensitivity, specificity and reproducibility in addition to a low intra and inter-observer variation.
▪ Most fertility laboratories will already at this stage of development be able to perform the test.
▪ It is cost effective compared to standard semen analysis and may save money if it is used early, because it may predict whether the doctor should refer the couple for IUI, IVF or ICSI.

Furthermore andrologists and gynaecologist request a new method for evaluating male fertility potential, because semen analysis using sperm count motility and morphology do not predict fertility outcome in a large percentage of cases.
The inventors found a highly significant difference. Thus, the method of the present invention is much more specific than test based on semen analyses of sperm count, motility and morphology.

Another aspect of the invention relates to the use of the present method together with other methods for predicting sperm quality such as but not limited to determination of sperm count, motility and/or morphology.

### The method

The present invention provides a method for predicting the fertility potential of a male.

One aspect of the invention relates to determining the expression level of CYP24A1. The expression level of CYP24A1 is used as a positive marker for sperm quality and thereby the male fertility potential.

Thus the present invention relates to a method according to claim 1.

### Fertility potential

A couple is normally considered to be infertile if the couple has not conceived after 12 months of contraceptive-free intercourse.

The fertility potential in a male mammal should be understood as the ability of said male's sperm to accomplish fertilization or to fertilize the egg. The male fertility potential is normally evaluated by semen analysis.

Thus the fertility potential in a male mammal depends on semen quality as semen quality correlates with the ability of semen to accomplish fertilization. Semen quality depends on both sperm quantity and quality. Decreased semen quality is a major factor of male infertility.

A semen analysis evaluates certain characteristics of a male's semen and the sperm contained in the semen. It may be done while investigating a couple's infertility. It is also used in stud farming, dog feeding and farm animal breeding. The characteristics measured by the current tests for semen analysis are only some of the factors in semen quality. The most common characteristics measured to evaluate sperm quality are: sperm count, motility, morphology, volume, fructose level and pH.

Thus the current method to predict the fertility potential in a male mammal is by measuring sperm count, motility and morphology. The World Health Organization (WHO) has until recently defined that a male individual has a normal fertility potential if the semen analysis shows a sperm number above 40 millions, more than 50% motile sperm and more than 12% morphological normal spermatozoa. WHO has, however, now changed this definition and lowered the reference values as a high proportion of the male population otherwise would be regarded as having a fertility potential below normal.

The semen analysis is however not an accurate estimate of male fertility potential and has been shown not always to correlate with the ability of the semen to accomplish fertilization or the ability of the semen to fertilize the egg.

### Male mammal

Reference to a "mammal", "subject" or a "individual" includes a human or non-human species including primates, livestock animals (e.g. sheep, cows, pigs, horses, donkey, goats), laboratory test animals (e.g. mice, rats, rabbits, guinea pigs, hamsters) and companion animals (e.g. dogs, cats). The present invention has applicability, therefore, in human medicine as well as having livestock and veterinary and wild life applications.

In a preferred embodiment the mammal is a human.

In a particular preferred embodiment the mammal is a man.

In another preferred embodiment the mammal is a livestock animal.

In preferred embodiment the mammal is selected from the list consisting of sheep, cows, pigs and horses.

### Semen sample

Semen is a mixture of an excreted fluid and cells. The fluid is also known as seminal fluid that usually contains spermatozoa. It is secreted by the gonads (sexual glands) and other sexual organs of male or hermaphroditic animals and may be able to fertilize female ova. In humans, seminal fluid may contain several components besides spermatozoa: proteolytic and other enzymes as well as fructose are elements of seminal fluid which promote the survival of spermatozoa and provide a medium through which they mature and get the ability to move or "swim". The process that results in the discharge of semen is called ejaculation.

In a preferred embodiment the semen sample is to be understood as a sample comprising semen and/or components derived from semen.

In a preferred embodiment the semen sample is a sample comprising spermatozoa. Rarely in the clinic subjects are seen with semen samples comprising very few or even no spermatozoa in their semen, samples from such a subjects is still of interest and thus included by the present definition of the sample base, as such samples would indeed be a sample which would be indicative of low fertility potential.

The semen sample may be obtained after ejaculation, aspiration from the testis, epididymis or after testicular biopsy or microdissection of the testis.

In the most preferred embodiment the semen sample is obtained after ejaculation.

In a particular preferred embodiment the semen sample is a sample comprising spermatozoa and/or components derived from a ejaculate.

Procedures for collecting semen samples from human or animals such as farm animals is well described in the literature and well known for a person skilled in the art.

In another embodiment of the present invention, a minimum of handling steps of the sample is necessary before measuring the expression level of the at least one protein of the vitamin D metabolizing machinery.

In the present context, the subject "handling steps" relates to any kind of pre-treatment of the semen sample before determining the expression level of the at least one protein of the vitamin D metabolizing machinery. Pre-treatment procedures includes washing, lysis, immunocapture, cytospin, fixation, separation, spin down, filtration, dilution, distillation, concentration, inactivation of interfering compounds, centrifugation, heating, fixation, addition of reagents, or chemical treatment.

In a preferred embodiment the semen sample is an ejaculate.

In one embodiment the ejaculate may be lysed, fixed or otherwise modified upon direct ejaculation in to a container containing chemicals enabling this.

In a preferred embodiment the sample may be up-concentrated by centrifugation using a gradient such as Percoll gradient centrifugation. In another preferred embodiment the sample may be up concentrated by centrifugation without a gradient.

In a presently preferred embodiment said pre-treatment procedures comprises mixing the semen sample with a phosphate buffered saline solution and subsequently spinning down the sample.

In a particular preferred embodiment the freshly delivered semen sample is centrifuged and cellular sediments collected before determining the expression level of the at least one protein of the vitamin D metabolizing machinery. The cellular sediment may be fixed using an appropriate fixative such as but not limited to ethanol or formaldehyde.

Sperm may be "washed" by density gradient centrifugation or by a "direct swim-up" technique that doesn't involve centrifugation.
In another embodiment the sperm are separated from the seminal fluid before determining the expression level of the at least one protein of the vitamin metabolizing machinery.

In a particular preferred embodiment no pre-treatment of the sample is necessary.

In a particular preferred embodiment the sample is a raw unmodified semen sample. The raw unmodified semen sample may be fresh.

One aspect of the present invention relates to a method wherein the semen sample may be stored for several days before determination of the expression level of the at least one protein of the vitamin D metabolizing machine. In a preferred embodiment pre-treatment of the samples such as but not limited to cytospin of the sample prolongs the time the sample can be stored.

In a preferred embodiment the samples may be stored for at least one day, such as at least 7 days such as at least 30 days. In another preferred embodiment the samples may be stored for several years before detecting the expression of the at least one protein of the vitamin D metabolism machinery such as at least one year, such as at least two years such as at least five years such as at least 10 years.

In one embodiment chemicals facilitating storage are added to the semen sample.

Thus, one embodiment of the present invention enables the male individual to make the semen sample in the privacy of his own home and subsequently sending the sample to laboratory for determining the fertility potential.

### Expression level

In one embodiment of the present invention the expression level is to be understood as the relative amount of spermatozoa in a semen sample that express the at least one protein of the vitamin D metabolizing machinery.

In another embodiment of the present invention the expression level is to be understood as absolute number of spermatozoa in a semen sample that express the at least one protein of the vitamin D metabolizing machinery.

In yet another embodiment of the present invention the expression level is to be understood as the fraction of spermatozoa in a semen sample that express the at least one protein of the vitamin D metabolizing machinery.

The presence or level of the at least one protein of vitamin D metabolising machinery may be determined at the level of the molecule itself or to the extent to which a gene is expressed. The level of the at least one protein of vitamin D metabolising machinery is measured by conventional analytical methods, such as immunological methods known to the art.

Measurements of the at least one protein of vitamin D metabolising machinery can be performed at gene, RNA, or protein level in accordance with the teachings herein.

As stated above, detection of the at least one protein of vitamin D metabolising machinery may be made at the protein or nucleic acid levels. Consequently, reference to presence or level of said at least one protein of vitamin D metabolising machinery includes direct and indirect data. For example, high levels of mRNA of CYP24A1 are indirect data showing high levels of CYP24A1. Ligands to the at least one protein of vitamin D metabolising machinery are particularly useful in detecting and/or quantization of these molecules.

The expression level of the at least one protein of the vitamin D metabolising enzyme as used herein refers to the absolute or relative amount of protein corresponding to this protein of the vitamin D metabolising machinery in a given sample. Thus, the expression level refers to the amount of protein in a sample. The expression level is usually detected using conventional detection methods.

In a preferred embodiment the expression level of at least one protein of the vitamin D metabolising machinery may be measured as the fraction of spermatozoa expressing said at least one protein of the vitamin D metabolising machinery. The expression level of the at least one protein of the vitamin D metabolising machinery may also be measured as a percentage of spermatozoa expressing said at least one protein of the vitamin D metabolising machinery. The absolute number of spermatozoa expressing the at least one protein of the vitamin D metabolising machinery may also be used.

In a particular preferred embodiment the expression level of the protein of the vitamin D metabolizing machinery refers to the percentage of spermatozoa with a distinct expression of said at least one protein of the vitamin D metabolising machinery. This may be measured using immunocytochemistry as described in the examples.

In another preferred embodiment the expression level of the protein of the vitamin D metabolizing machinery refers to the total protein level of the at least one protein of the vitamin D metabolizing machinery in a semen sample.

### Vitamin D metabolizing machinery

Vitamin D has widespread biological functions, among these an essential role for calcium homeostasis. The biological effects of vitamin D rely on activation of cholecalciferol (vitamin D₃), which normally starts in the skin, where 7-hydrocholesterol is converted to vitamin D₃ after UVB radiation, and subsequently is activated by the hepatic 25-hydroxylases (CYP2R1, CYP27A1) and the renal 1α-hydroxylase (CYP27B1). The active 1,25(OH)₂D₃ (calcitriol) binds with high affinity to the VDR and elicits its effect, until it is inactivated by CYP24A1.

Recently is was shown by Jensen et al that the VDR, activating (CYP2R1, CYP27A1, CYP27B1) and inactivating (CYP24A1) enzymes are expressed in human germ cells, epithelium of the epididymis, prostate, seminal vesicle and in mature human spermatozoa, where VDR and the metabolising enzymes co-localise in the postacrosomal region, neck and midpiece (Jensen et al 2009).

The present invention relates to a method to predict the fertility potential in a male mammal comprising the steps of providing a semen sample from said male mammal and subsequent determining the expression level of CYP24A1.

The vitamin D receptor (VDR), also known as the calcitriol receptor, is a member of the nuclear receptor family of transcription factors. Upon activation by vitamin D, the VDR forms a heterodimer with the retinoid-X receptor and binds to hormone response elements on DNA resulting in expression or transrepression of specific geneproducts. In humans, the vitamin D receptor is encoded by the VDR gene.

CYP24A1, also known as 1,25-dihydroxyvitamin D(3) 24-hydroxylase, is an enzyme that in humans is encoded by the CYP24A1 gene. This gene encodes a member of the cytochrome P450 superfamily of enzymes. The cytochrome P450 proteins are monooxygenases that catalyze many reactions involved in drug metabolism and synthesis of cholesterol, steroids and other lipids. This protein initiates the degradation of 1,25-dihydroxyvitamin D3, the physiologically active form of vitamin D3, by hydroxylation of the side chain to form calcitroic acid.

CYP27A1 is cytochrome P450 oxidase involved in the metabolism of vitamin D3.

CYP27B1, also known as 25-Hydroxyvitamin D3 1-alpha-hydroxylase, is a cytochrome P450 enzyme that catalyzes the hydroxylation of calcidiol to calcitriol.

CYP2R1, also known as vitamin D 25-hydroxylase is an enzyme that in humans is encoded by the CYP2R1 gene. CYP2R1 is a member of the cytochrome P450 superfamily of enzymes. This enzyme is a microsomal vitamin D hydroxylase that converts vitamin D into calcidiol.

According to the present invention the levels of 1, 2, 3, 4 or 5 proteins of the vitamin D metabolizing machinery are determined. Preferable the level of 1 or 2 or 3 or 4 proteins of the vitamin D metabolizing machinery are determined. Most preferable the levels of 1 or 2 proteins of the vitamin D metabolizing machinery are determined.

In yet at more preferable embodiment the level of one protein of the vitamin D metabolizing machinery is determined.

Thus in an preferred embodiment the level of at least 1, at least 2, at least 3 or at least 4 proteins of the vitamin D metabolizing machinery are determined. Most preferable the levels of at least 1 protein of the vitamin D metabolizing machinery are determined.

Most preferable the levels of 1-2 proteins of the vitamin D metabolizing machinery are determined, or the level of 1-3 proteins of the vitamin D metabolizing machinery, or the level of 1-4 proteins of the vitamin D metabolizing machinery.

### Reference level

The present invention relates to a method according to claim 1.

In order to determine the fertility potential, means for evaluating the detectable signal of the at least one protein of the vitamin D metabolising machinery measured involves a reference or reference means. The reference also makes it possible to count in assay and method variations, kit variations, handling variations and other variations not related directly or indirectly to the level of the at least one protein of the vitamin D metabolising machinery.

In the context of the present invention, the term "reference" relates to a standard in relation to quantity, quality or type, against which other values or characteristics can be compared, such as e.g. a standard curve.

The reference data reflects the semen expression level obtained from already clinically diagnosed subject identified by standard clinical means such as but not limited to sperm count, motility and morphology. Thus a reference by comparison of the at least one protein of the vitamin metabolising machinery for male mammals with a fertility potential below normal and/or the semen expression level of the at least one protein of the vitamin metabolising machinery for male mammals with a normal fertility potential is trivial for the person skilled in the art. As will be generally understood by those of skill in the art, methods for screening for reduced fertility potentials are processes of decision making by comparison. For any decision making process, reference values based on male mammals having a fertility potential below normal and/or male mammals having a fertility potential below normal are needed.

In the present invention the reference values are the expression levels of the measured at least one protein of the vitamin D metabolising machinery, for example, CYP24A1, in male mammals with normal fertility and male mammals with a fertility below normal. A set of reference data is established by collecting the reference values for a number of samples. As will be obvious to those of skill in the art, the set of reference data will improve by including increasing numbers of reference values.

In the context of the present invention it is possible to determine a reference level based on the proportion of the spermatozoa that expresses at least one protein of the vitamin D metabolizing machinery, such as but not limited to CYP24A1, in semen samples from male mammals with a normal fertility potential or a fertility potential below normal.

In one embodiment of the present invention it is possible to determine a reference level by investigating the abundance of the at least one protein of the vitamin D metabolizing machinery in the semen samples from male mammals with a normal fertility potential or a fertility potential below normal. This may be determined by a simple test such as ELISA or immunocytochemistry.

To determine a reference level for an ELISA test the expression level of the protein in semen samples may be determined from patients who already have been investigated for the expression of at least one protein of the vitamin D metabolizing machinery, for instance CYP24A1, using immunocytochemistry as described in the examples. Thus, the fraction of spermatozoa expressing CYP24A1 in said samples are known. Based on these results a standard curve may be obtained that shows the relationship between the protein level detected by ELISA and the percentage of spermatozoa that express CYP24A1. The standard curve can thereby be used to determine the amount of protein, which corresponds to for instance that at least 3% of the spermatozoa express CYP24A1.

The same approach for determining a reference level applies to all the proteins of the vitamin D metabolising machinery.

The same approach may be applied for other techniques as known in the art.

In one preferred embodiment of the present invention, the reference means is an internal reference means and/or an external reference means.

In the present context the term "internal reference means" relates to a reference which is not handled by the user directly for each determination, but which is incorporated into a device for the determination of the concentration of the at least one protein of the vitamin D metabolising machinery, whereby only the 'final result' or the final measurement' is presented. The terms the "final result" or the "final measurement" relate to the result presented to the user when the reference value has been taken into account.

In a further embodiment of the present invention, the internal reference means is provided in connection to a device used for the determination of the concentration of the at least one protein of the vitamin D metabolising machinery.

In yet an embodiment of the present invention the device is selected from the group consisting of an assay, a stick, a dry-stick, an electrical device, an electrode, a reader (spectrophotometric readers, IR-readers, isotopic readers and similar readers), histochemistry, and similar means incorporating a reference.

In the present context, the term "external reference means" relates to a reference which is handled directly by the user in order to determine the concentration of the at least one protein of the vitamin D metabolising enzyme, before obtaining the 'final result' or the 'final measurement'.

In yet a further embodiment of the present invention external reference means are selected from the group consisting of a table, a diagram and similar reference means where the user can compare the measured signal to the selected reference means. The external reference means relates to a reference used as a calibration, value reference, information object, etc. for CYP24A1 and which has been excluded from the device used.

One embodiment of the present invention relates to a method according to the present invention, wherein said reference level/predetermined value is indicative of a normal fertility potential of said individual.

One embodiment of the present invention relates to a method according to the present invention, wherein said reference level/predetermined value is indicative of a fertility potential below normal of said individual.

Although any of the known analytical methods for measuring the semen level of the at least one protein of the vitamin D metabolising machinery will function in the present invention, as obvious to one skilled in the art, the analytical method used for said protein must be the same method used to generate the reference data for said protein. If a new analytical method is used for the at least one protein of the vitamin D metabolising machinery, a new set of reference data, based on data developed with the method, must be generated. Thus, the technique utilized to analyze the semen should be the same for the reference data and the samples to be screened.

### Risk Assessment

The present inventors have successfully developed a new method to predict the fertility potential in a male mammal. The results presented in the examples shows that CYP24A1 appears to be an efficient marker for the fertility potential in a male mammal. The discrimination is better than with conventional semen analysis.

To determine whether the subject has an increased risk of having fertility potential below normal, a cut-off must be established. This cut-off may be established by the laboratory, the physician or on a case-by-case basis by each subject.

The cut-off level could be established using a number of methods, including: percentiles, mean plus or minus standard deviation(s); multiples of median value; patient specific risk or other methods known to those who are skilled in the art.

The multivariate discriminant analysis and other risk assessments can be performed on the commercially available computer program statistical package Statistical Analysis system (manufactured and sold by SAS Institute Inc.) or by other methods of multivariate statistical analysis or other statistical software packages or screening software known to those skilled in the art.

As obvious to one skilled in the art, in any of the embodiments discussed above, changing the risk cut-off level of a positive or using different a priori risks which may apply to different subgroups in the population, could change the results of the discriminant analysis for each patient.

When expression levels of a specific protein in a semen sample are compared to a reference level they can either be different (above or below the reference value) or equal. However, using today's detection techniques is an exact definition of different or equal result can be difficult because of noise and variations in obtained expression levels from different samples. Hence, the usual method for evaluating whether two or more expression levels are different or equal involves statistics.

Statistics enables evaluation of significantly different expression levels and significantly equal expressions levels. Statistical methods involve applying a function/statistical algorithm to a set of data. Statistical theory defines a statistic as a function of a sample where the function itself is independent of the sample's distribution: the term is used both for the function and for the value of the function on a given sample. Commonly used statistical tests or methods applied to a data set include t-test, f-test or even more advanced test and methods of comparing data. Using such a test or methods enables a conclusion of whether two or more samples are significantly different or significantly equal.

The significance may be determined by the standard statistical methodology known by the person skilled in the art.

In a preferred embodiment the reference level should be understood as at least 3% of the spermatozoa expressing CYP24A1. Thus, a semen sample from a male mammal wherein at least 3% of the spermatozoa express CYP24A1 indicates that said male mammal has a normal fertility potential.

In another preferred embodiment the reference level should be understood as at least 3% of the spermatozoa expressing CYP24A1, such as at least 4%, such as at least 5%, such as at least 6%, such as at least 7%, such as at least 8%, such as at least 9%, such as at least 10%, such as at least 11%, such as at least 12%, such as at least 13%, such as at least 14%, or such as at least 15%.

When more than 3%, such as 4%, such as 5% or such as 6% of the spermatozoa in a semen sample from a male mammal express the at least one protein of the vitamin D metabolizing enzymes it indicates that said male mammal has a normal fertility potential.

Another preferred embodiment relates to a method wherein the male mammal is considered to have a normal fertility potential if the abundance of the expressed protein of the vitamin D metabolizing machinery correlates with expression of said protein in more than 3% of the spermatozoa, such as more than 4% of the spermatozoa, such as more than 5% of the spermatozoa, such as more than 6% of the spermatozoa, such as more than 7% of the spermatozoa, such as more than 8% of the spermatozoa, such as more than 9% of the spermatozoa, such as more than 10% of the spermatozoa, such as more than 11% of the spermatozoa, such as more than more than 12% of the spermatozoa, such as more than 13% of the spermatozoa, such as more than 14% of the spermatozoa, such as more than 15% of the spermatozoa.

A preferred embodiment relates to a method wherein the male mammal is considered to have a normal fertility potential if the abundance of the expressed protein of the vitamin D metabolizing machinery correlates with expression of said protein in more than 3% of the spermatozoa.

In the presently most preferred embodiment the reference level should be understood as the amount of protein corresponding to that a fraction above 3% of spermatozoa express said protein.

The chosen reference level may be changed depending on the mammal for which the test is applied.

The chosen reference level may be changed if desiring a different specificity or sensitivity as known in the art.

### Sensitivity

As used herein the sensitivity refers to the measures of the proportion of actual positives which are correctly identified as such - in analogy with a diagnostic test, i.e. the percentage of mammals or people having a fertility potential below normal who are identified as having a fertility potential below normal.

Usually the sensitivity of a test can be described as the proportion of true positives of the total number with the target disorder i.e. a fertility potential below normal. All patients with the target disorder are the sum of (detected) true positives (TP) and (undetected) false negatives (FN).

### Specificity

As used herein the specificity refers to measures of the proportion of negatives which are correctly identified - i.e. the percentage of mammal with a normal fertility potential that are identified as not having a fertility potential below normal. The ideal diagnostic test is a test that has 100 % specificity, i.e. only detects mammal with a fertility potential below normal and therefore no false positive results, and 100 % sensitivity, and i.e. detects all mammals with a fertility potential below normal and therefore no false negative results.

For any test, there is usually a trade-off between each measure. For example in a manufacturing setting in which one is testing for faults, one may be willing to risk discarding functioning components (low specificity), in order to increase the chance of identifying nearly all faulty components (high sensitivity). This trade-off can be represented graphically using a ROC curve.

Selecting a sensitivity and specificity it is possible to obtain the optimal outcome in a detection method. In determining the discriminating value distinguishing mammals having a fertility potential below normal, the person skilled in the art has to predetermine the level of specificity. The ideal diagnostic test is a test that has 100% specificity, i.e. only detects mammals with a fertility potential below normal and therefore no false positive results, and 100% sensitivity, and i.e. detects all mammals with a fertility potential below normal and therefore no false negative results. However, due to biological diversity no method can be expected to have 100% sensitive without including a substantial number of false negative results.

The chosen specificity determines the percentage of false positive cases that can be accepted in a given study/population and by a given institution. By decreasing specificity an increase in sensitivity is achieved. One example is a specificity of 95% that will result in a 5% rate of false positive cases. With a given prevalence of 1% of e.g. a fertility potential below normal in a screening population, a 95% specificity means that 5 individuals will undergo further physical examination in order to detect one (1) fertility potential below normal if the sensitivity of the test is 100%.

As will be generally understood by those skilled in the art, methods for screening for reduced fertility potentials are processes of decision making and therefore the chosen specificity and sensitivity depends on what is considered to be the optimal outcome by a given institution/clinical personnel.

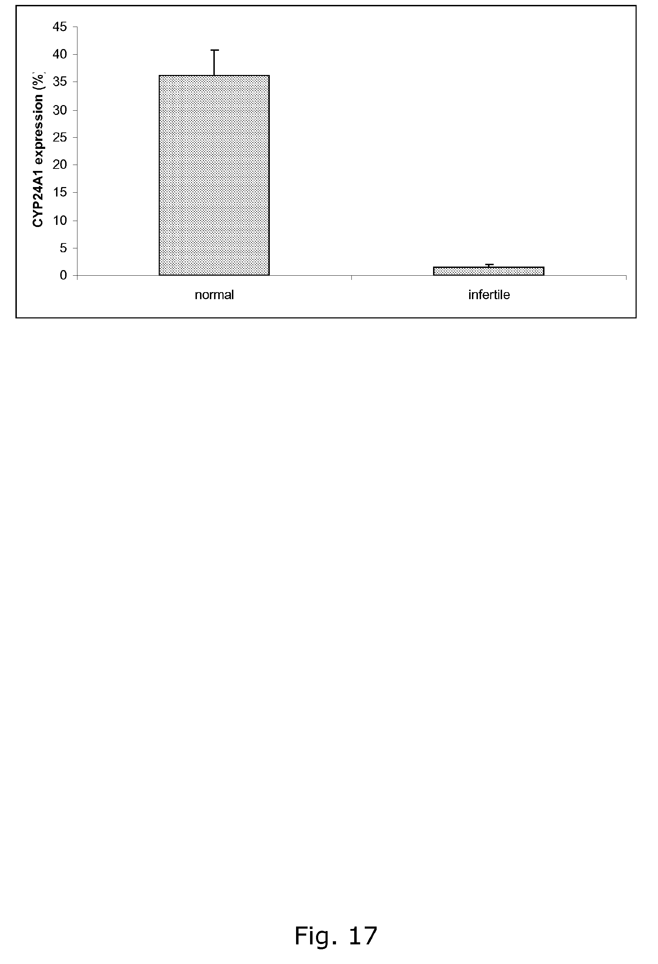

The present invention relates to a method with a high specificity. This is exemplified by but limited to the examples herein. In the example sub-fertile and normal men in the examples give a sensitivity of 67.9%, specificity 70.2% and the chance of being normal (ppv-estimate) is 97,7%, if the test is used in the general population and > 3% of the spermatozoa express CYP24A1 at the annulus. When using the groups of true sub-fertile and normal men (normospermic sub-fertile plus normal men) in the examples give a sensitivity of 73.4%, specificity 94.8% and the chance of being normal (ppv-estimate) is 99.6%, if the test is used in the general population and > 3% of the spermatozoa express CYP24A1 at the annulus. We have now extended these studies and have investigated further 390 men, of which 302 were infertile (mean 1,4) and 88 were normal (mean 36,1). We found a highly significant difference in the expression of CYP24A1 between these groups. This clearly shows that expression of these proteins are markers for male fertility potential (Figure 17).

It would be obvious for a person skilled in the art that it may be advantageous to select a higher specificity and/sensitivity in some cases and in other cases a lower specificity and/or sensitivity.

In a preferred embodiment the invention relates to a method with a high specificity such as at least 60%, such as at least 70%, such as at least 80%, such as at least 90% such as at least 95% such as 100%.

In another preferred embodiment the invention relates to a method with a high sensitivity such as at least 60% such as at least 70% such as at least 80% such as at least 90% such as 100%.

The cut-off level could be established using a number of methods, including: percentiles; mean plus or minus standard deviation(s); multiples of median value; patient specific risk or other methods known to those who are skilled in the art.

Another aspect of the invention relates to a method wherein the ratio between at least two proteins of the vitamin D metabolising machinery is used to predict whether or not a male mammal has a normal fertility potential.

Thus, in an embodiment of the present invention the expression level of CYP27A1 is determined and compared with the reference level which correspond to a chosen level of CYP24A1.

### Location

One aspect of the present invention relates to a method wherein the expression of CYP24A1 is detected on at least one spermatozoa present in the semen sample.

The sperm cell consists of a head, a midpiece and a tail. The head contains the nucleus with densely coiled chromatin fibres, surrounded anteriorly by an acrosome, which contains enzymes used for penetrating the female egg. The post-acrosomal region is located caudal for the acrosome and ends in the neck, which marks the transition to the midpiece. The midpiece has a central filamentous core with many mitochondria spiralled around it, used for ATP production for the journey through the female cervix, uterus and uterine tubes. The tail or "flagellum" executes the lashing movements that propel the spermatozoa. The annulus is a septin-based ring structure located at the junction of the midpiece and the principal piece of spermatozoa flagellum.

A particular preferred embodiment relates to a method wherein the expression of the least one protein of the vitamin D metabolizing machinery is detected at the post-acrosomal region, neck, midpiece and/or at the annulus.

Another preferred embodiment relates to a method wherein the expression of the least one protein of the vitamin D metabolizing machinery is detected at least at one of the locations on the sperm cells selected from the group consisting of the post-acrosomal region, neck, midpiece and the annulus.

In another preferred embodiment the expression of the least one protein of the vitamin D metabolizing machinery is detected at the neck and the annulus.

A presently preferred embodiment relates to a method wherein expression of the least one protein of the vitamin D metabolizing machinery is determined at the annulus.

### Measuring Techniques

The presence or expression level of the protein of the vitamin D metabolising machinery molecule may be determined at the level of the molecule itself or to the extent to which a gene is expressed.

A particular embodiment of the present invention relates to a method the expression level is determined by measuring the level of mRNA and/or protein.

The level of the proteins of the vitamin D metabolising machinery such as but not limited to CYP24A1 is measured by conventional analytical methods, such as immunological methods known to the art.

It is to be understood that any of the methods described in the present invention is platform independent. Accordingly, any immunological method such as but not limited to ELISA, Luminex, immunohistochemistry, immunocytochemistry, Multiplex, FACS, ImageStream, western blotting, Immunoblotting, TRF-assaýs, immunochromatographic lateral flow assays, Enzyme Multiplied Immunoassay Techniques, RAST test, Radioimmunoassays, immunofluorescence and immunological dry stick assays (e.g. cromatographic stick test) may be applicable to the present invention.

Thus a preferred embodiment of the present invention relates to a method wherein the determination of the at least one protein level is performed using a method selected from the group consisting of immunohistochemistry, immunocytochemistry, FACS, ImageStream, Western Blotting, qPCR, RT-PCR, qRT-PCR, ELISA, Luminex, Multiplex, Immunoblotting, TRF-assaýs, immunochromatographic lateral flow assays, Enzyme Multiplied Immunoassay Techniques, RAST test, Radioimmunoassays, immunofluorescence and immunological dry stick assays.

As stated above, detection of the proteins of the vitamin D metabolising machinery may be made at the protein or nucleic acid levels. Consequently, reference to presence or level of said proteins of the vitamin D metabolising machinery includes direct and indirect data. For example, high levels of CYP24A1 mRNA are indirect data showing high levels of CYP24A1.

In a most preferred embodiment the proteins of the vitamin D metabolising machinery is determined at the protein level.

Ligands to the proteins of the vitamin D metabolising machinery are particularly useful in detecting and/or quantifying these proteins.
Antibodies to the proteins of the vitamin D metabolising machinery are particularly useful. Techniques for the methods contemplated herein are known in the art and include, for example, sandwich assays, xMAP multiplexing, Luminex, ELISA and ELISpot. Reference to antibodies includes parts of antibodies, mammalinized (e.g. humanized) antibodies, recombinant or synthetic antibodies and hybrid and single chain antibodies.
Both polyclonal and monoclonal antibodies are obtainable by immunization with the immune signalling molecules or antigenic fragments thereof and either type is utilizable for immunoassays. The methods of obtaining both types of sera are well known in the art.
Polyclonal sera are less preferred but are relatively easily prepared by injection of a suitable laboratory animal with an effective amount of the immune signalling molecule, or antigenic part thereof, collecting serum or plasma from the animal and isolating specific sera by any of the known immuno-adsorbent techniques. Although antibodies produced by this method are utilizable in virtually any type of immunoassay, they are generally less favoured because of the potential heterogeneity of the product.

The use of monoclonal antibodies in an immunoassay is particularly preferred because of the ability to produce them in large quantities and the homogeneity of the product. The preparation of hybridoma cell lines for monoclonal antibody production derived by fusing an immortal cell line and lymphocytes sensitized against the immunogenic preparation can be done by techniques, which are well known to those who are skilled in the art.

Several techniques are known to the skilled addressee for determination of biological markers such as CYP24A1.

In a presently preferred embodiment the invention relates to a method where the determination is performed by immunohistochemistry.

### Immunohistochemistry

Immunohistochemistry refers to the process of localizing antigens e.g. proteins in cells of a tissue section exploiting the principle of antibodies binding specifically to antigens in biological tissues. In the procedure, depending on the purpose and the thickness of the experimental sample, either thin (about 4-40 µm) slices are taken of the tissue of interest, or if the tissue is not very thick and is penetrable it is used whole. The slicing is usually accomplished through the use of a microtome, and slices are mounted on slides. There are two strategies used for the immunohistochemical detection of antigens in tissue, the direct method and the indirect method. In both cases, many antigens also need an additional step for unmasking, which often makes the difference between staining and no staining. Unlike immunocytochemistry, the tissue does not need to be permeabilized because this has already been accomplished by the microtome blade during sample preparation. Detergents like Triton X-100 are generally used in immunohistochemistry to reduce surface tension, allowing less reagent to be used to achieve better and more even coverage of the sample.

In another presently preferred embodiment the invention relates to a method where the determination is performed by immunocytochemistry.

### Immunocytochemistry

Immunocytochemistry refers to the process of localizing antigens e.g. proteins in cells collected in a suspension/fluid exploiting the principle of antibodies binding specifically to antigens. The procedure depends on the type and amount of cells in question, but usually the cells in the suspension/fluid is fixed to a microscope glass and subsequently treated as immunohistochemical slides. Special systems may be used to transfer the cells but not the fluid to a hard support material that can be analysed. Special reagents, which facilitate permeabilization may be applied.

One particular aspect of the present invention relates to a method that can classify CYP24A1 expression at the annulus (separates the midpiece from the tail) of mature spermatozoa, based on an antibody that recognizes CYP24A1 at the annulus. A computer system or at least one independent observer may then calculate the percentage of spermatozoa expressing CYP24A1 at the annulus. Alternatively assessing the expression of CYP27A1, CYP2R1, CYP27B1 and VDR at the neck to distinguish sperm from normal and infertile men.

### Elisa

Immunoassays, in their most simple and direct sense, are binding assays. Antibody binding to for instance CYP24A1 can be detected by any immunoassay means known in the art. Preferably, antibody binding is detected by an assay selected from the group consisting of protein microarray assay, radioimmunoassay (RIA), enzyme-linked immunosorbent assay (ELISA), fluoroimmunoassay, immunofluorometric assay, and immunoradiometric assay.

Most preferably, antibody binding is detected by ELISA.

Thus a preferred embodiment relates to a method wherein the determination of the expression of the at least one protein of the vitamin D metabolizing machinery is performed by ELISA.

Enzyme-linked immunosorbent assay, also called ELISA, enzyme immunoassay or EIA, is a biochemical technique used mainly in immunology to detect the presence of an antibody or an antigen in a sample. In simple terms, in ELISA, an unknown amount of antigen is affixed to a surface, and then a specific antibody is washed over the surface so that it can bind to the antigen. This antibody is linked to an enzyme, and in the final step a substance is added that the enzyme can convert to some detectable signal. Thus in the case of fluorescence ELISA, when light of the appropriate wavelength is shone upon the sample, any antigen/antibody complexes will fluoresce so that the amount of antigen in the sample can be inferred through the magnitude of the fluorescence.

### Combination markers

Measurements of at least one of the proteins of the vitamin D metabolising machinery can be combined with measurements of other markers at gene, RNA, or protein level in accordance with the teachings herein.

One aspect of the invention relates to a method wherein measurement of CYP24A1 is combined with a disease specific marker.

Another aspect of the invention relates to a method wherein measurement of CYP24A1 is combined with a sperm specific marker.

This invention may be used in combination with diagnostic test for cancer such testis cancer. Thus a third aspect of the invention relates to a method wherein measurement of CYP24A1 is combined with a cancer specific marker.

A particular preferred embodiment relates to a method wherein the expression level is combined with expression level of at least one protein selected from each of the following groups: annulus markers such as the Septin family (1-12). DNA packaging: Protamin 1, Protamin 2 and histones. Oncogenes and growth receptors: FGFR1OP1, FGFR1OP2, FGFR1, FGFR2, FGFR3 and related proteins. Another preferred embodiment relates to a method wherein the expression level is combined with expression level of Septin 1-12.

A preferred embodiment relates to a method wherein the expression level is combined with marker relevant for chromatin packaging and DNA quality such as protamin1, protamin 2, acridine orange, DNA fregmentation, SCSA, TUNEL, monobromobimane (mBBr),chromomycin A3 (CMA3) etc.
Another preferred embodiment relates to a method wherein the expression level is combined with expression level of vitamin d regulated genes such as Calbindin, calreticulin, TRPV6, TRPV5, NCX, osteocalcin, osteopontin, aromatase, klotho. Another preferred embodiment relates to a method wherein the expression level is combined with expression level of other ion channels or pump such as T and L-type calcium calcium channels, CatSper, PMCA, SERCA, SEPCA, Na-K ATPase, klotho, carbonic anhydrase.

A preferred embodiment relates to a method wherein the expression level is combined with expression level of at least one protein selected from the group consisting of centrosomal markers such as, growthfactors insulin, insulin receptor, prolactin receptor, markers of acrosome, mitochondrial function or viability such as acrosin, propidium iodide, ethidium homodimer, carboxyfluorescein diacetate, SYBR-14 nucleic acid, 7-amino-actinomycin-D, Merocyanine 540 and other cyanine dyes, CD46, Pisum sativum agglutinin, Arachis hypogaea agglutinin, other agglutinins, 5,5,6,6-tetrachloro-1,1,3,3-tetraethylbenzimidazolylcarbocyanine Iodide (JC-1), 3,3-dihexyloxacarbocyanine iodide, Chloromethyl-X-rosamine, mitotracker dyes (green, red, deep red etc.), 2,7-bis-(2-carboxyethyl)-5-(and-6)-carboxyfluorescein and other markers for pH, acetoxymethyl ester, ACR2, CTC, Gluthathione reductase, free thiol-groups, valinomycin, all phosphotyrosines, all markers for Apoptosis such as annexin V, YO-PRO-1 iodide, carboxy SNARF-1 AM, all caspases etc., All markers for oxidative stress and thus reactive oxygenspecies (ROS) such as 2,7-dichlorodihydrofluorescein diacetate (H2DCFDA), CMH2DCFDA (5-(and 6-)chloromethyl-2,7-dichlorodihydrofluorescein diacetate, acetyl ester), Dihydroethidium (DHE), MitoSOX red, 4,5-diaminofluorescein diacetate (DAF-2 DA), BODIPY 581591 C11, BODIPY 665/676, 5-iodoacetamidofluoresceine (5-IAF).
A presently preferred embodiment of the invention relates to a method according to claim 10.

Semen quality does not predict if the sperm can bind to the egg, but studies have shown that it is only the high quality sperm, which are able to complete the transportation to the fallopian tube where it meets and fertilize the egg.

In a preferred embodiment this method may be used in combination with a sperm-egg binding test where it is determined whether or not the sperm are able to bind to the egg and undergo acrosome reaction.

This invention will add valuable information to the diagnosis of male infertility, because it may predict whether the sperm quality is sufficient to achieve pregnancy spontaneously or if the couple should proceed to assisted reproduction or further investigation.

Thus a particular preferred embodiment of the invention the prediction of the fertility potential will predict whether the sperm quality is sufficient to achieve pregnancy spontaneously or if the couple should proceed to assisted reproduction or further investigation.

Today, some fertility clinics evaluate semen quality by using a CASA (electronic detection) system, which is able to count the number of motile spermatozoa in fresh semen samples. There exists no test on the market that is able to discriminate spermatozoa with normal and abnormal morphology.
The inventors have shown that CYP24A1 expression is significantly correlated with morphology (p<0.004), motility (p<0.002), and total sperm number (p<0.0005) and can be conducted on old samples. Additionally, this test has classified several patients as sub-fertile, who solemnly by routine semen analysis would be regarded as normal men. These men have been classified as sub-fertile based on our test results, which was supported by low expression of other biological markers such as Septin 4 and reinforced by the anamnesis of the infertile couple and measurements of inhibin B and FSH. Furthermore, the inventors validate their results in a larger cohort of normal and infertile men (Figure 17)

Furthermore the expression of at least one protein of the vitamin D metabolising machinery such as but not limited to CYP24A1 can be used in a test for deciding which assisted reproductive techniques (mild insemination, IVF or ICSI) is preferable. For instance if the method described herein predict that a man has a normal fertility potential it means that his sperm will be suitable for any method of assisted reproductive techniques, if the woman has no obvious fertility problems insemination will be the first choice of intervention. If the woman has infertility causes the couple should proceed to IVF. If the method described herein predicts that a man has a fertility potential below normal his sperm will not be suitable for insemination and the couple should probably proceed directly to ICSI.

### Brief description of the figures

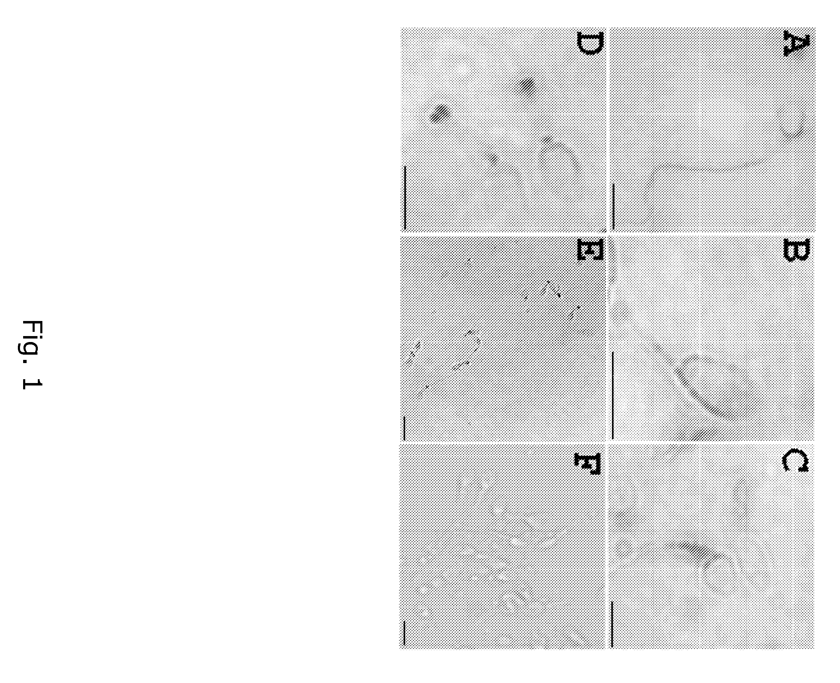

Figure 1 show immunocytochemical expression of VDR, VD metabolising enzymes and septin 4 in human spermatozoa. A: CYP2R1 expression in neck and tail. B: expression of CYP27B1 in postacrosomal region and neck. C: VDR expression in neck and midpiece. D: CYP24A1 in the neck and at the annulus. E; Septin 4 expression at the annulus. F: Negative control. Bar corresponds to 10 µm.

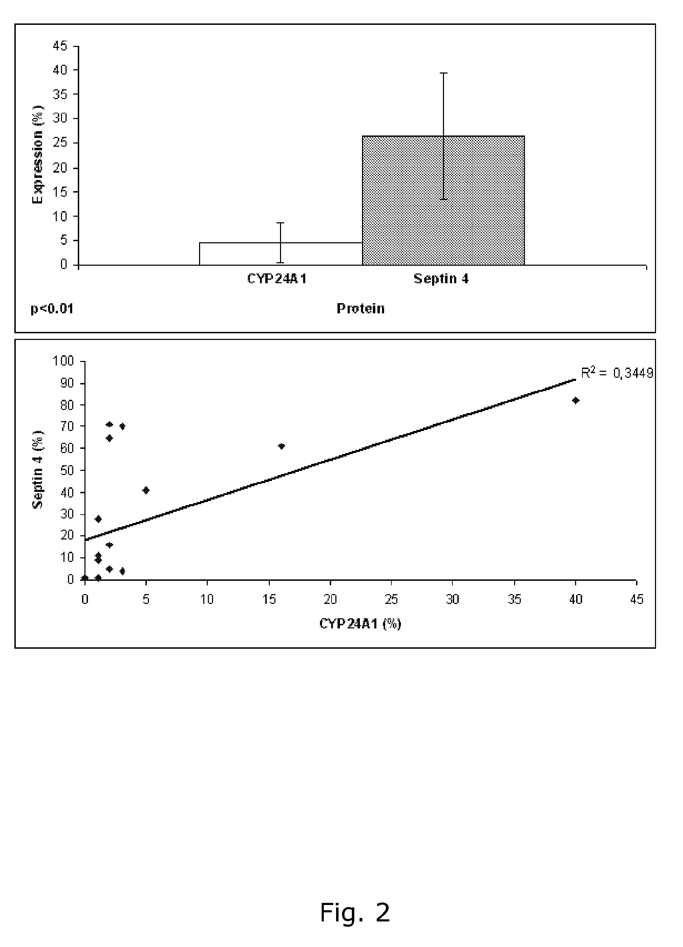

Figure 2 shows CYP24A1 and Septin 4 expression in spermatozoa from 20 men. A: mean expression and error bars indicates 95% CI. B: correlation between septin 4 and CYP24A1 expression.

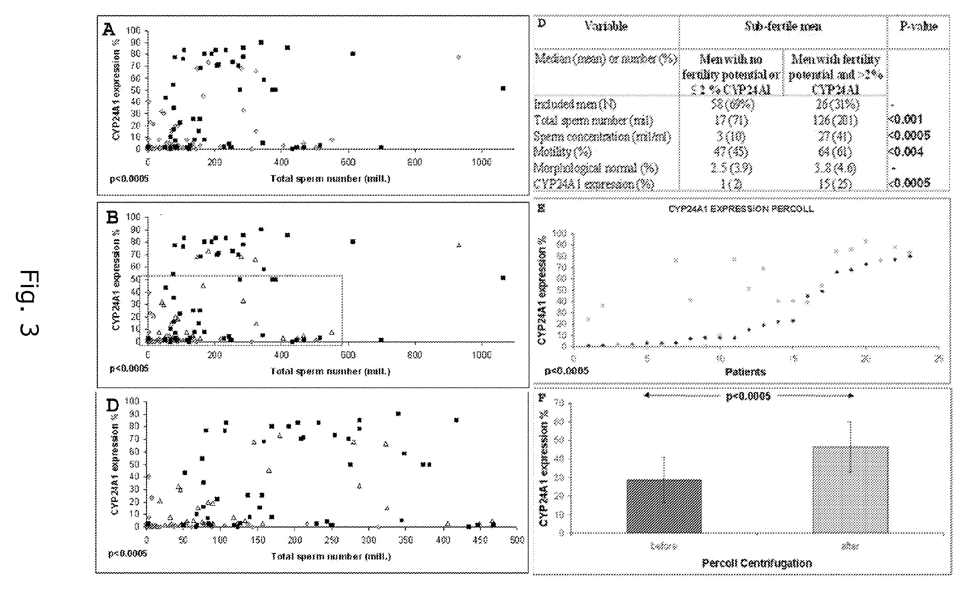

Figure 3 shows percentage of spermatozoa expressing CYP24A1 at the annulus. A-C Military conscripts (normal men) are marked with gray squares and subfertile men are marked with dark diamonds. Sub-fertile men with normal total sperm number, normal sperm motility (normospermic sub-fertile) are in B and C marked with bright dots. The boxed region in Fig.3B is enlarged in Fig. 3C. D: Baseline characteristics of the sub-fertile men with low and high expression of CYP24A1.E: CYP24A1 expression before (dark gray) and after (bright gray) Percoll centrifugation. F: Mean CYP24A1 expression before and after Percoll centrifugation, error bars indicates 95 CI.
Figure 4: CYP24A1 expression and semen quality variables. Patients are stratified according to: A: total sperm count, B: sperm concentration, C: sperm motility (ABC), D: progressive sperm motility (AB). E: sperm morphology. Results in A-e are mean and error bars indicate 95% CI. Correlations coefficients and p-value are listed for each variable. F: Enrichment of CYP24A1 positive spermatozoa in individual semen samples after percoll gradient centrifugation. Thick horizontal lines indicate median values before (15%) and after (41%) percoll gradient centrifugation.

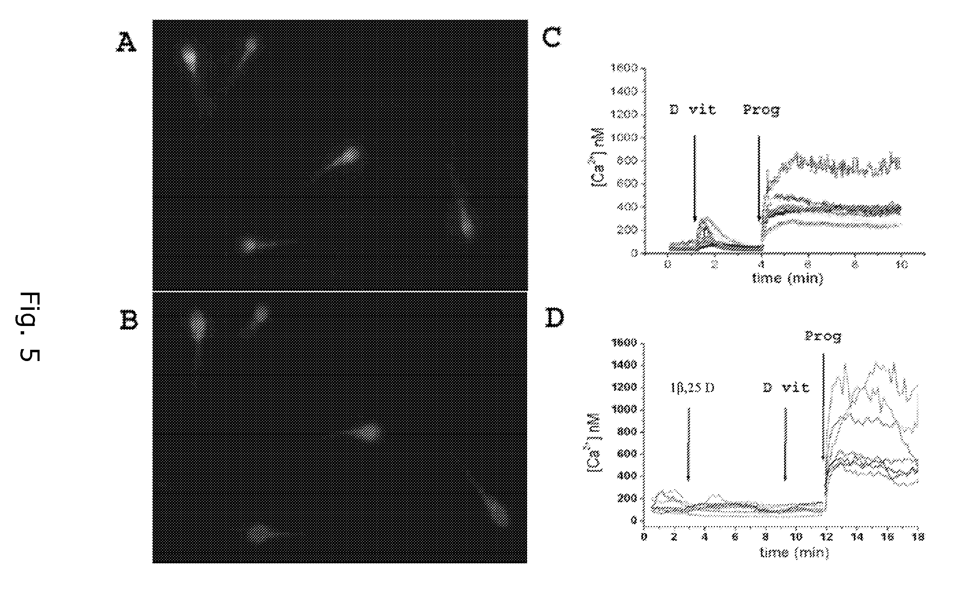

Figure 5 shows intracellular calcium levels in human spermatozoa. A: Fura 2 loaded spermatozoa, where color/grayscale indicates calcium level. B: immediate change in calcium levels after addition of 1nM 1,25D₃. C: Corresponding changes in intracellular calcium after addition of 1nM 1,25D₃ and 10µM progesteron. D: Effect of 1β,25D₃, 1,25D₃ and 10µM progesterone.

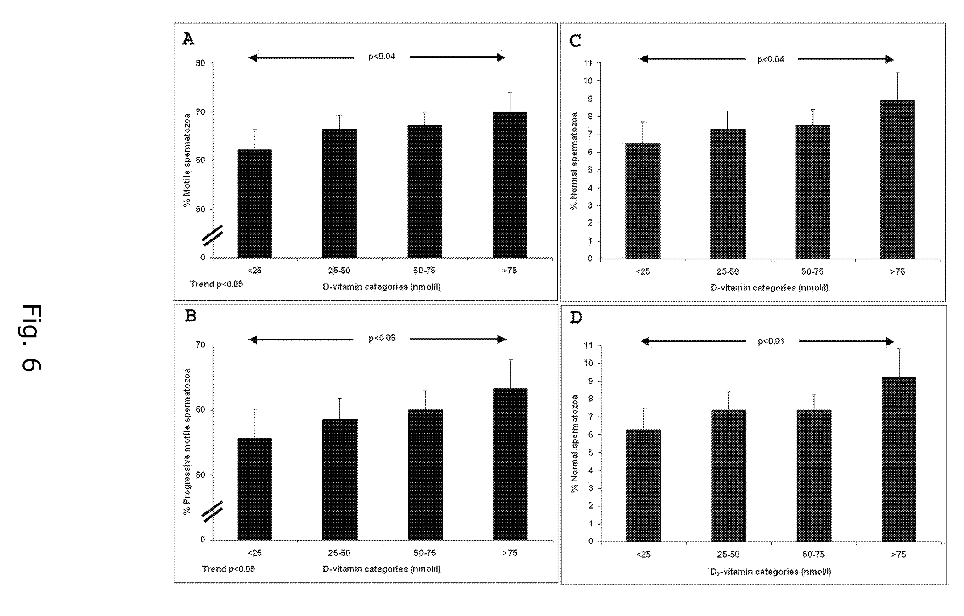

Figure 6 shows correlation between serum levels of 25-OH D₂₊₃ and sperm quality variables. A: Association with sperm motility (ABC). B: Association with progressive motile spermatozoa (AB). C: Normal sperm morphology. D: Morphology and Vitamin D3. Geometrical mean and error bars indicate 95% C.I. Note separated Y-axis in A and B.
Figure 7 shows group characteristics for immunocytochemical evaluation of CYP24A1 expression in human spermatozoa. * 7 men with azoospermia were excluded.
Figure 8 shows characteristics of the "normospermic sub-fertile" men with >2 % spermatozoa expressing CYP24A1. Classification of all infertile couples, where the men had >2 % of their spermatozoa expressing CYP24A1 at the annulus.* marks potentially primary testis failure, with low sperm quality, relatively low inhibin B and high FSH, N.A: not available, PCOS: polycystic ovarian failure, DM: Diabetes Mellitus, IBD: Inflammatory Bowel Disease, MCTD: Mixed connective tissue disease, WPW: Wolff Parkinson White, S.O: salphingo oophororectomy, ALS: amyotrofic lateral schlerosis, ab. Pro: abortus provocatus.

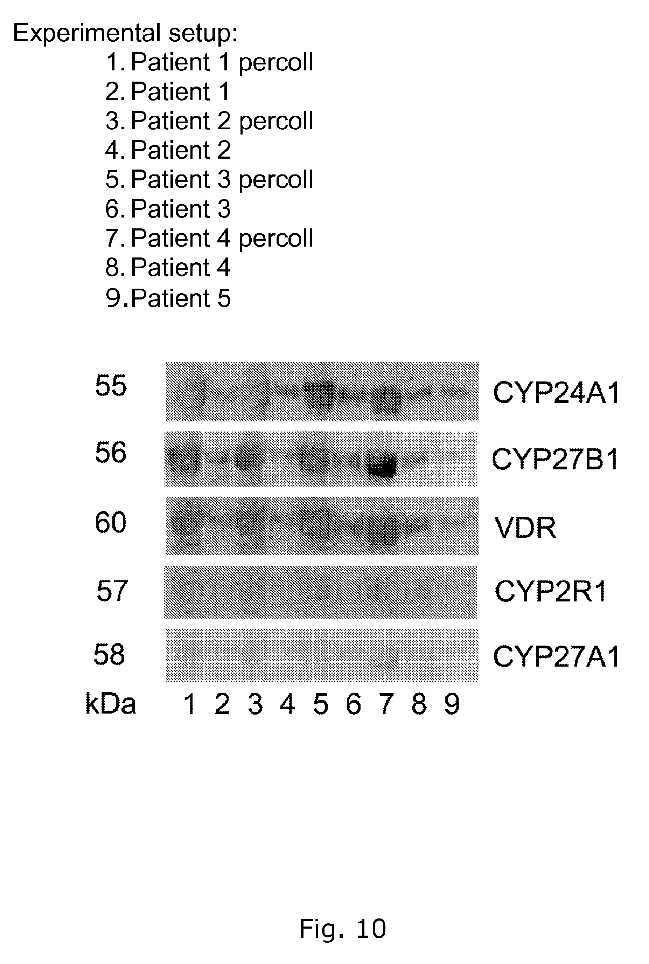

Figure 10 shows Western blot performed on sperm samples before and after percoll centrifugation. We investigated expression of VDR, CYP27B1, CYP2R1, CYP27A1 and CYP24A1 in semen samples from 5 men, where 4 of the samples (1-8) were investigated before and after percoll centrifugation.

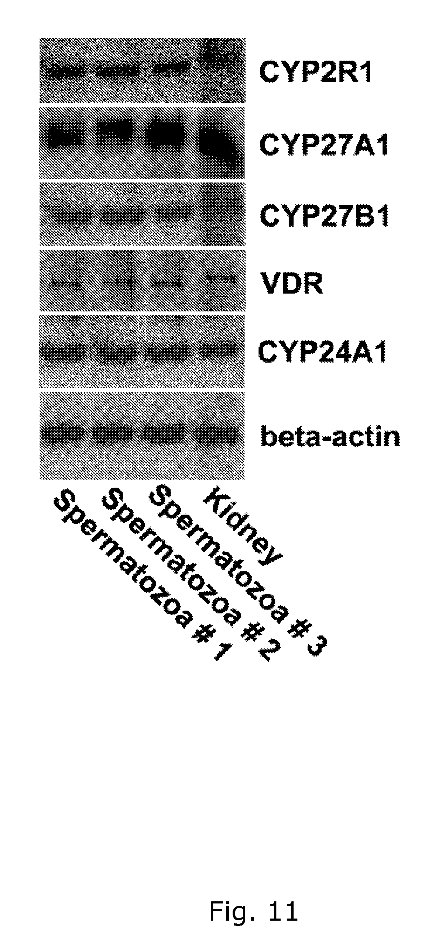

Figure 11 shows Western Blot showing the presence of VDR and all the metabolizing enzymes in human spermatozoa.

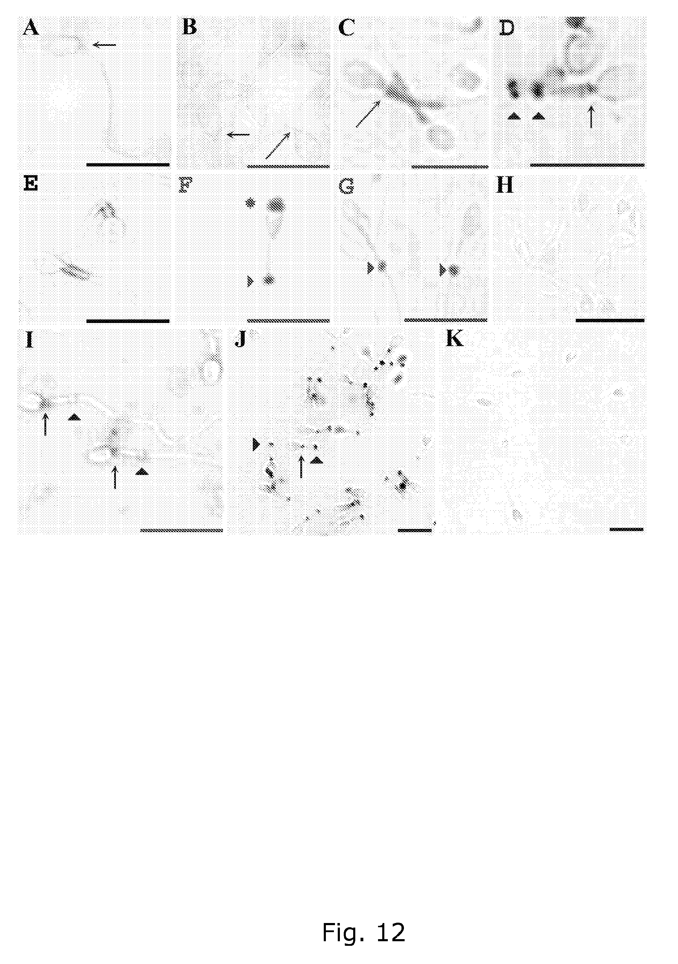

Figure 12 shows immunocytochemical expression of VDR and the VD metabolizing enzymes in human spermatozoa. A: CYP2R1 expression. B: CYP27B1 expression. C: VDR expression. D: CYP24A1 expression. E: CYP24A1 expression in midpiece. F: CYP24A1 expression in cytoplasmic droplet. G: Septin 4 expression. H: No primary antibody (negative control). I: double staining with VDR (red color in neck) and CYP24A1 (yellow-brown color at the annulus). J: Normal semen sample (sperm concentration 123 mill/ml, motility 87%) with 85% spermatozoa expressing CYP24A1 at the annulus. K: Spermatozoa from an infertile man (sperm concentration 6 mill/ml, motility 50%) with 0% expressing CYP24A1 at the annulus. Arrowhead indicates expression at the annulus, arrows indicate expression at the neck and asterix expression in cytoplasmic droplets in the head. Bar corresponds to 10 µm.
Figure 13 shows quantitative data showing that spermatozoa from normal men also have a higher expression of VDR, CYP27B1 and CYP2R1 in addition to CYP24A1. Figure A: showing mean VDR expression in the neck of spermatozoa from Normal (N=34) and infertile men (N=35). Error bars indicate s.e.m. Figure B: showing mean CYP2R1 expression in the neck of spermatozoa from Normal (N=4) and infertile men (N=9). Error bars indicate s.e.m. Figure C: showing mean CYP27B1 expression in the neck of spermatozoa from Normal (N=9) and infertile men (N=12). Error bars indicate s.e.m
Figure 14 shows the difference in expression is supported by functional studies showing that fewer sperm from infertile men responds to calcitriol and it is unable to increase motility in infertile men compared with a significant increase in normal men. Vitamin D and intracellular calcium in human spermatozoa. A: Fura 2 loaded spermatozoa. Color indicates intracellular calcium level (low to high: bright to dark). B: Rapid change in intracellular calcium levels after addition of 1 nM 1,25(OH)2D3, arrowhead indicates one unresponsive spermatozoa C: Corresponding changes in intracellular calcium after addition of 10 µM progesterone, arrowhead indicates the spermatozoa being unresponsive to 1 nM 1,25(OH)2D3. D: Increase in intracellular calcium following treatment with 1 nM 1,25(OH)2D3 and 10 µM progesterone. Each trace represents calcium levels in single spermatozoa and arrows indicate the spermatozoa being unresponsive to 1,25(OH)2D3 (black trace). E: Induction of sperm motility after treatment with 1 nM 1,25(OH)2D3 in normal and subfertile men. Horizontal bars indicate mean.

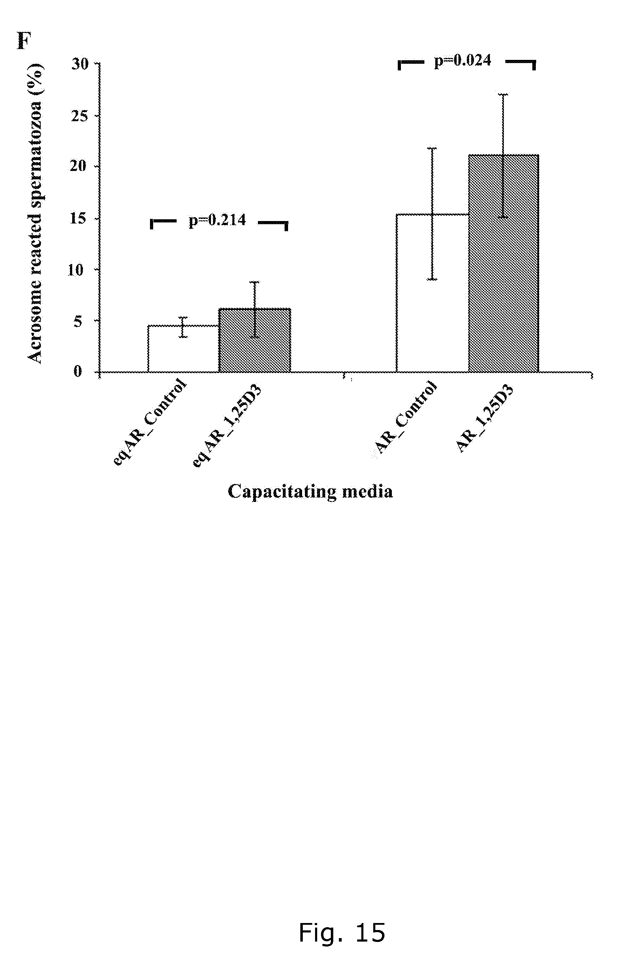

Figure 15 shows induction of the acrosome reaction in capacitating media following treatment with control (DMSO) or 1nM activated VD (1,25(OH)2D3).
   The percentage of acrosome reacted spermatozoa following treatment with control (AR_control) and activated VD (AR_1,25D3) and the percentage with an equatorial expression (eqAR_control/1,25D3).

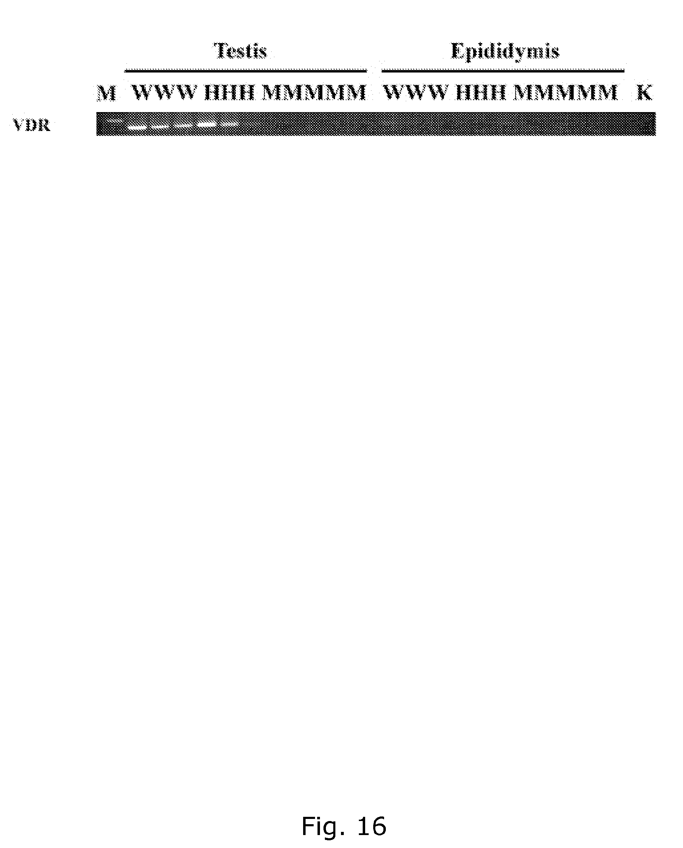

Figure 16 shows RT-PCR from testis and epididymis from wildtype and VDR knock out Mice. M: marker, W: +/+ wildtype, H: +/- Heterozygotes, M: -/- homozygotes mutants, K: control no cDNA.
Figure 17 shows new quantitative data that spermatozoa from normal men have a higher expression of CYP24A1. Figure A: showing mean CYP24A1 expression in the neck of spermatozoa from Normal (N=88) and infertile men (N=302). Error bars indicate s.e.m.

The present invention will now be described in more details in the following.

### Examples

### Example 1

### Introduction

Vitamin D (VD) has widespread biological functions, among these an essential role for calcium homeostasis. The biological effects of VD rely on activation of cholecalciferol (vitamin D₃), which normally starts in the skin, where 7-hydrocholesterol is converted to VD₃ after UVB radiation, and subsequently is activated by the hepatic 25-hydroxylases (CYP2R1, CYP27A1) and the renal 1α-hydroxylase (CYP27B1). The active 1,25(OH)₂D₃ (calcitriol) binds with high affinity to the vitamin D receptor (VDR) and elicits its effect, until it is inactivated by CYP24A1.

We have previously shown that VDR, and all the VD metabolising enzymes are expressed in the male ejaculatory duct, germ cells and mature spermatozoa. Expression of VDR and the VD metabolising enzymes co-localise in the neck and midpiece of human spermatozoa indicating a role of VD on sperm quality.

Beneficial effects of VD on reproductive function have been demonstrated in several animal studies. In rodents, VD deficiency results in reduced sperm counts and lower fertility rates in females inseminated with semen from VD deficient males. This is reinforced by the observed male infertility in VDR knock-out mice.

Generally,1,25(OH)₂D₃ elicits both genomic and rapid non-genomic actions through binding to VDR. A rapid non-genomic effect is expected in the mature and transcriptionally silent spermatozoa, and VD has rapid non-genomic effects in other mesenchymal derived tissue, where 1,25(OH)₂D₃ through second messengers such as cAMP and IP3 or directly via ion-channels increases intracellular calcium levels.

VDR and the VD metabolising enzymes are co-expressed in the neck of human spermatozoa, where the IP₃ gated calcium storage (nuclear redundant envelope) has been described as well. The plausible effect of VD through calcium signalling is highlighted by animal studies, where both fertility and semen quality were restored in VD deficient rodents after normalization of calcium levels, while the impaired fertility in VDR knock-out mice only partly could be corrected by calcium supplementation. The calcium concentration in the epididymal and prostatic fluid is 2-3 fold higher than in serum, and the high extracellular calcium is regarded important for sperm maturation and capacitation in both the male and female reproductive tract.

Our initial investigation revealed that VDR, CYP27B1 and CYP24A1 were expressed in a comparable proportion of spermatozoa from each individual. Due to the distinct expression pattern of CYP24A1 at the annulus and the transcriptional induction of CYP24A1 by 1,25(OH)₂D₃, we choose CYP24A1 as the best marker of VD-metabolism in spermatozoa from sub-fertile and normal men. We present here the results of this investigation, supported by functional studies revealing that 1,25(OH)₂D₃ increases calcium in human spermatozoa, and a cross sectional study of 300 normal young men showing an association of VD serum levels and semen quality variables.

### Material and methods

### Ethics

All sub-fertile men were recruited from the andrology clinic at University Department of Growth and Reproduction at Rigshospitalet, Denmark in accordance with the Helsinki Declaration and after approval from the local ethics committee (permit no. KF 01 2006-3472). The normal men were recruited from an ongoing surveillance program of semen quality among young Danish men from the general population, approved by the Danish National Committee on Biomedical Research Ethics, Copenhagen Region.

### CYP24A1 expression study

### Study populations

In total 144 men were screened in this part of the study; 91 sub-fertile men and 53 men from the general population. In the 91 sub-fertile group 7 had azoospermia and were therefore excluded. The sub-fertile men were all patients referred for semen analysis at the andrology section of the University Department of Growth and Reproduction (dept. GR) at Rigshospitalet in Denmark.

Of the 84 sub-fertile men included in this study, 77 where referred for investigation because of involuntary infertility for at least 1 year, and the remaining 7 for control of their semen quality due to unilateral orchidectomy (2), medication (2), hypogonadotrop hypogonadism (2), and haemospermia. All sub-fertile men delivered two semen samples, had a physical and andrological examination performed and had a blood sample taken for evaluation of reproductive hormone levels (described in the cross-sectional study), karyotyping and evaluation of microdeletions on the Y-chromosome.

Except for a minor subgroup (18) of the sub-fertile men, who delivered the semen sample at dept. GR, but went through clinical investigation and blood samples in a fertility clinic in Copenhagen. Patient files were obtained from this fertility clinic as well as from the fertility clinics where the couples subsequent to the clinical evaluation were referred to for assisted reproduction. Journals were only obtained after consent from the patients.

The 53 young men were consecutive participants in an ongoing study of semen quality of Danish men from the general population. All young Danish men, except for those suffering from chronic severe diseases, are required to attend a compulsory medical examination when they are 18-19 years before they may be considered for military service. Therefore, such a group of men can be considered representative of the general population of young men, and in collaboration with the military health authorities we enrolled men to a surveillance study.

They went through a physical examination, body weight and height of the participants were assessed and a full andrological examination was conducted including measurement of testicular size measured by orchidometer and ultrasound of both testis. Prior to the examination the men answered a comprehensive questionnaire, and on the day of examination they delivered one semen sample. The men received 500 DKK (approximately 67 Euro) for their participation in the study.

### Semen analysis

The protocol for semen analysis was the same for the 144 men, who was included for investigation of CYP24A1 expression, and the 300 normal men included in the cross sectional study. All participants produced a semen sample by masturbation and 393 out of 444 men had at least 48 hours of ejaculation abstinence. The sample was collected in a wide mouthed plastic container. A detailed description of assessment of semen samples has previously been described.

Semen volume was estimated by weighing the collection tube with the semen sample and subtracting the predetermined weight of the empty tube, assuming that 1 ml = 1 g. Phase-contrast microscopy (positive phase-contrast optics) was used for the examination of fresh semen. For sperm motility assessment, 10 µl of well-mixed semen was placed on a clean glass slide that had been kept at 37 °C and covered with a 22x22 mm coverslip.

The preparation was placed on the heating stage of a microscope at 37 °C and immediately examined at x400 magnification counting 200 spermatozoa. The sperm were classified as progressive motile (WHO class A+B), non-progressive motile (class C) or immotile (class D). The motility counting was repeated on a second aliquot of 10µl well-mixed semen.

For the assessment of sperm concentration, each semen sample was thoroughly mixed for at least 10 min in a rotation device. An aliquot of the sample was put into the diluent using a positive displacement pipette and mixed for a further 10 min. The diluent consisted of 50 g NaHCO3, 10 ml 40% formaldehyde and distilled water up to 1 litre. The sperm concentration was subsequently assessed using a Burker-Turk haemocytometer.

One drop of the diluted specimen was transferred to each chamber of the haemocytometer, which was allowed to stand for 5 min in a humid chamber before the cells were counted at a total microscope magnification of x 400. Only spermatozoa with tails were counted. Smears were air dried, fixed in 96% ethanol and Papanicolaou stained for a morphology analysis according to strict criteria.

### Immunocytochemistry (IHC)

Ejaculates from all men (double blinded so all with azoospermia was also evaluated) were stained immunocytochemically for CYP24A1 and some were investiagated for the remaining proteins by IHC and WB. Briefly, freshly delivered semen samples were centrifuged (5 min at 1500rpm) and cellular sediments were collected, after routine semen analysis had been performed. A semen sample from each patient was diluted if necessary, divided in portions of 100µl, centrifuged on a microscope slide with 400 µl phosphate-buffered saline buffer and dried. Fixation with ethanol, formalin or no-fixation gave the same results. Antigen retrieval was accomplished by microwaving the sections for 15 min in TEG buffer (Tris6.06 g, EGTA 0.95 g in 5 l, pH 9.0). Sections were washed in 2% hydrogenperoxid to block endogenous peroxidase.

Afterwards, all sections were incubated with 2% non-immune goat serum or mouse serum (Zymed Histostain kit, San Francisco,CA, USA) in Tris buffered saline (TBS) to minimize cross-reactivity. Primary antibodies were purchased from Santa Cruz Biotechnology Inc., Santa Cruz, CA [CYP2R1 (C-15) sc-48985, CYP27A1 (P-17) sc-14835, CYP27B1 (H-90) sc-67261, VDR (H-81):sc-9164, CYP24 (H-87): sc-66851], Septin 4 (H-120) sc-20179. All antibodies were tested by western blotting and have been validated in the normal human kidney (Jensen et al., 2010) . Initial experiments were carried out with different antibody dilutions (1:50 to 1:500), different buffers (TEG, citrate, Urea) and with different fixatives (formalin, ethanol and none).

The optimal dilutions of the primary antibodies were: CYP2R1 1:100, CYP27A1 1:200, CYP27B1 1:200, VDR 1:50 and CYP24 1:200. After 16 h of incubation at 4° C, the sections were incubated with biotinylated goat anti-rabbit IgG (Zymed Histostain kit) or biotinylated donkey antigoat IgG (1:400), before a peroxidase-conjugated streptavidin complex (Zymed Histostain kit) was used as a tertiary layer. Visualization was performed with amino ethyl carbasole (Zymed Histostain kit).The slides were washed with TBS between incubation steps.

Two independent investigators evaluated all slides (), while another was in charge of both blinding and preparation of the slides. The slides were evaluated by an initial general overview, before a representative area was carefully examined and the exact percentage of spermatozoa with the distinct expression of CYP24A1 at the annulus was determined. A concomitant expression in the neck did not exclude the spermatozoa from being positive.

The evaluation of the slides was highly reproducible. 390 samples were conducted in duplicates throughout the investigation with reproducible results. However, 8% of the samples were scored markedly different between the two observers. The discrepancy was often influenced by methodological problems. These slides were reanalyzed by the two observers simultaneously and scored. The data presented here is the mean of the two observers score. The initial 80 samples were co-stained with VDR to ensure that the expression pattern of the receptor followed the percentage of sperm expressing CYP24A1.

Semen from 23 randomly selected men was used for additional studies. Initially part of the semen was investigated as described above, before the remaining semen sample was percoll centrifugated, and another cytospin was made for comparison with the cytospin made prior to percoll centrifugation. This investigation was performed to determine whether CYP24A1 is a positive predictive marker of semen quality, because a percoll density gradient separates high quality sperm from the remnants of the semen sample and the percentage of spermatozoa expressing CYP24A1 is supposed to be higher after percoll separation.

### Functional studies on human spermatozoa

The intracellular free calcium concentration ([Ca2+]i) was measured in human spermatozoa from 12 normal men. Freshly delivered semen samples went through percoll density gradient centrifugation to optimize the number of spermatozoa expressing VDR and the metabolising enzymes. Spermatozoa were diluted in 1ml Krebs-Ringer buffer and the cells were loaded with 3 µM fura-2/AM (Invitrogen, USA) in medium 1640 RPMI for 30 min and washed with Krebs-Ringer buffer. Subsequently 80µl sperm content were diluted in 920 µl Krebs-Ringer buffer and were loaded on polynysine-coated chambers to minimize movement of the vital spermatozoa (Lab-Tek; Nalge Nunc International, USA).

Images were acquired by means of a Zeiss Axiovert 135 microscope equipped with a Zeiss Achrostigmat 40 x 1.3 NA objective. Excitation was obtained by a Polychrome V illuminator from Till Photonics (Germany) and images were acquired using a Cool Snap CCD camera (Photometrics, USA) from Robert Scientific (Malaysia). For measurements of [Ca2+]i, the excitation wavelengths were 338 and 380 nm, measuring emission above 510 nm using a cut-off filter.

Calculations of [Ca²⁺]i were conducted by MetaFluor software from Molecular Devices and using a Kd of 160 nM. After background subtraction, ratio images were formed using MetaFluor software. 1,25-(OH)₂D₃, 25-OH D₃ and ionomycin were purchased from Sigma-Aldrich (USA), while the PLC-inhibitor U73122 (1-[6-[[17fi-3-methoxyestra-1,3,5(10)-trien-17-yl]amino]hexyl]-1H-pyrrole-2,5-dione) was from Biomol, PA, U.S.A. VDR antagonists, non-genomic 1 β,25-(OH)₂D₃ was a kind gift from Tore Duvold (LEO Pharma Denmark) and genomic ZK 159222 a kind gift from Dr. Andreas Steinmeyer (Bayer Schering).

### Reproductive hormones and biochemical analyses

A blood sample was drawn from the cubital vein, centrifuged and the serum separated and frozen. Reproductive hormone levels were assessed at the University Dept GR. Serum levels of follicle stimulating hormone (FSH), was determined using a time-resolved immunofluorometric assay (Delfia; Wallac, Turku, Finland). The level of Inhibin-B was determined by a specific two-sided enzyme linked immunoassay (Bio-Innovation Ltd, Oxford, UK).

All hormone assessments were performed at the end of the study to reduce the influence of inter-assay variations. The intra- and inter-assay coefficients of variation (CV) for measurement of FSH were 3 and 4.5% respectively and for inhibin-B 15 and 18%. P-Albumine (Cat.nr. 04657357 190, CV% inter assay: 1.2 - 1.8 %), P-Calciumtotal (Kat.nr. 04718933 190, CV% inter assay : 0.6 - 1.2 %), and P-Alkaline Phosphatase (Kat.nr. 04657373 190, CV% inter assay : 0.6 - 1.3 %) were measured on Cobas c501 from Roche Diagnostics A/S, and P-Parathyreoidea hormone (Cat.nr. 11972103 122, CV% inter assay : 2.5 - 3.4 %) was measured on Cobas e601. The manufacturer supplied reagents and calibrators. All procedures and methods were followed as recommended by the manufacturer (Roche). Albumin corrected calcium = P-Calcium - 0,0012 (P-Albumin - 644) mmol/l.

### Vitamin D

25-OH Vitamin D2 (25-hydroxyergocalciferol Mw 412.7) was purchased from Fluka (prod. No. 17937), and 25-OH Vitamin D3 (25-hydroxycholecalciferol Mw 400.7) was from Sigma-Aldrich (prod. No. H 4014). [2H6]-25-OH Vitamin D3 (26,26,26,27,27,27-hexadeutero-25-hydroxycholecalciferol, Mw: 406.7) from Synthetica A/S (Oslo Research Park. Gaustadalleen 21, Oslo, Norway), dissolved in 50% ethanol/acetic acid, 200 mmol/l, pH 4.0 was used as internal standard. As primary calibrator we used the Vitamin D standard from NIST (National Institute of Standards and Technology, USA, Product no. SRM 972), and as daily working calibrators, 25-OH Vitamin D2 and 25- OH Vitamin D3 added to human serum to obtain four calibrators with D2 concentration up to 200 nmol/l, and D3 concentration up to 300 nmol/l.

25-OH Vitamin D3 and 25-OH Vitamin D2 were analysed using isotope-dilution liquid chromato-graphy tandem mass spectrometry after liquid-liquid extraction. Internal standard (300 µl) was added to serum, control or calibrator (300 µl), and vortex-mixed for 2 minutes with 1 ml n-heptane. After mixing, the heptane phase was transferred to a heating block, and evaporated to dryness at 75°C under a stream of nitrogen. The residue was dissolved in 300 µl MeOH:H2O (80:20), and applied to the LC-MS/MS instrument (sample vol. 50 µl).

The LC-MS/MS system consisted of Waters Alliance 2795 HPLC interfaced to Waters Micromass Quattro Micro API tandem quadrupole mass spectrometer (Waters, Milford, MA). Chromatographic separation was achieved with a Waters analytical column (Atlantis dC18, 3 µm, 2.1 x 50 mm, part no. 186001291) equilibrated with MeOH:H2O (80:20) containing 0.1% formic acid. Applying a linear gradient of 100 % MeOH containing 0.1% formic acid over a time period of 3 minutes eluted the Vitamin D metabolites. Flow rate was 0.4 ml/min, and total cycle time on LC-MS/MS was 8 minutes.

The tandem mass spectrometer used positive electrospray ionization at an operating voltage of 3.5 kV, and a desolvation temperature of 350°C. To detect and quantify the Vitamin D metabolites, the instrument was operated in MRM (multiple-reaction monitoring) mode, with the following transitions : m/z+ 413.15/395.2 for 25-OH Vitamin D2, m/z+ 401.15/383.15 for 25-OH Vitamin D3, and m/z+ 407.2/389.3 for [2H6]-25-OH Vitamin D3. The interassay CVs for 25-OH Vitamin D3 were 2.2% and 2.8% at 30 and 180 nmol/l, respectively, and for 25-OH Vitamin D2 were 7.6% and 4.6 % at 43 and 150 nmol/l, respectively.

### Statistical analysis

Groups characteristics where compared using af non-parametric Mann-Whitney test. Results were verified using other tests including a t-test. Bivariate associations were investigated using the non-parametric Spearman correlation. Person correlation and Kandall's tau confirmed the results.

### Results

### CYP24A1 expression

We conducted immunocytochemical (ICC) experiments on 13 patients to ensure that CYP24A1 expression at the annulus was concomitant with expression of CYP2R1, CYP27A1, CYP27B1 and VDR (Figure 1). CYP27B1 and VDR were expressed in a similar proportion of spermatozoa, while CYP24A1 was generally expressed in a lower proportion of cells compared to CYP27B1 and VDR. In contrast, CYP2R1 was expressed in more spermatozoa, while CYP27A1 expression was inconsistent.

The ICC was followed by western blots (WB) performed on both fresh semen samples and on sperm after a modified percoll separation (55% gradient). WB showed that VDR, CYP27B1, CYP2R1, CYP27A1 and CYP24A1 were abundantly expressed in semen samples from both normal and infertile men, and we were able to detect substantial difference in the abundance of the investigated proteins following percoll seperation of high quality spermatozoa(Figure 10). To investigate whether expression of CYP24A1 at the annulus was similar to other known annulus markers, we evaluated 20 semen samples for both Septin 4 and CYP24A1 expression. We found that the expression was significantly (p<0.01) different between the annulus marker Septin 4 and CYP24A1. Septin 4 was expressed in a higher proportion of spermatozoa compared to CYP24A1, although there was a strong positive correlation between the expressions of both proteins (figure 2).

We initially included ICC analysis on spermatozoa from 137 men and the descriptive statistics of the subjects included in this part of the study are summarized in Figure 7. The proportion of spermatozoa expressing CYP24A1 at the annulus in the sub-fertile group was 1% (median) and 9% (mean) and in the normal young men 25% (median) and 36% (mean), which was significantly different (p<0.0005) (Figure 3). Semen quality differed significantly between the sub-fertile group (N=84) and the young men (N=53) from the general population.

After evaluating CYP24A1 expression at the annulus in the two groups, we analyzed CYP24A1 expression in relation to semen quality variables including sperm number, motility and morphology (figure 4). We found a significant association (p<0.0005) to total sperm count, sperm concentration (p<0.0005), motility (p<0.002), and morphology (p<0.004). However, when we analysed the association to semen variables in the two independent groups, where the respective values for the sub-fertile men to total sperm count (p<0.0005), concentration (p<0.0005), motility (p<0.06), while the trends for morphology and all the variables in the normal group were in-significant (figure 4A-F). Despite the significant difference in CYP24A1 expression between the normal and sub-fertile men, some of the men in the infertile group expressed CYP24A1 in a higher percentage (>2%) of spermatozoa.

### Thirty men from the sub-fertile group had > 2% spermatozoa expressing

CYP24A1, and 26 (87%) had relatively high sperm numbers, good sperm motility, high inhibin B, low FSH and an anamnesis, which indicated that they might have a fertility potential that resembled the men for the normal group (figure 8). In > 50 % of the cases their spouses had female infertility factors, some men were responsible for earlier pregnancies and the remaining were in many cases referred for other reasons than male infertility (figure 2). We compared these 26 men with the remaining 58 sub-fertile men with ≤ 2% expressing CYP24A1 and found a significantly higher sperm number (p<0.001), concentration (p<0.0005), motility (p<0.004) in the group of 26 men, while the observed trend for better morphology was insignificant (Figure 3D). Their semen characteristics and the correlation between CYP24A1 expression with semen quality variables resembled more the group of normal men than the remaining sub-fertile men and we named them "normospermic sub-fertile" (Figure 3 B-D and figure 42A-F).

Four out of the 30 men with >2 % expressing CYP24A1 could have a primary testis problem with low sperm counts in addition to many immotile and abnormal spermatozoa, but Inhibin- B levels were in only 2 out of these 30 men below 100 pg/ml and none of them had an Inhibin- B < 50 pg/ml. The medical history of the 4 men revealed the presence of reversible causes such as infectious disease or salazopyrin treatment prior to semen analysis, and in two cases the men were part of a couple with complicating female infertility factors. Another person had amyotrophic lateral sclerosis and was responsible for a pregnancy earlier in life, while only one had a known male fertility problem, which could have been aggravated by several years' treatment with immunosuppressive drugs (Figure 2).

We found 15 men from the healthy control group (N=53), who had < 3% spermatozoa with CYP24A1 expression. These men did not have any obvious andrological problems, but 13 men (87%) had a sperm concentration below 70 mill/m, 7 (47%) had a sperm concentration below 25 mill/ml, while motility and morphology was comparable with the sub-fertile men with high CYP24A1 expression. We noticed that some men with good semen quality, who had low expression of CYP24A1, additionally expressed Septin 4 in a low proportion (<10%) of spermatozoa.

To determine whether CYP24A1 expression could be a positive predictive marker for semen quality, we analyzed CYP24A1 expression in semen samples from 23 men before and after their sample went through a percoll density gradient centrifugation. The percentage of spermatozoa expressing CYP24A1 were higher in 17/23 (74%) patients after percoll centrifugation, and the percentage was either identical or the decline was very small in the remaining six patients (Figure 2E).

Comparing the results before and after percoll centrifugation showed a significant difference (p<0.0005) with higher CYP24A1 expression after percoll centrifugation. Furthermore, the variance was higher after percoll (non significant P=0.097), which indicated a better separation of individuals after percoll (Figure 3F). We only measured serum levels of VD in 26 of the infertile and 45 of the normal men, but there was no significant association with serum levels and CYP24A1 expression in the spermatozoa. Although we noticed a tendency towards high serum levels of VD and good semen quality (number, motility, morphology), it was not significant for any of the semen variables. CYP24A1 expression at the annulus may thus serve as a marker to distinguish normal and sub-fertile men in the general population.

This immunocytochemical test had a sensitivity of 67.9%, specificity was 70.2% and the chance of being normal (ppv-estimate, when applying the test in the general population) was 97.7% (95% CI: 96.3% - 98.7%), if > 3% of the spermatozoa expressed CYP24A1.We classified 26 out of the 30 sub-fertile men with ≥ 3 % spermatozoa expressing CYP24A1 as normospermic sub-fertile (Figure 8), which further increased the sensitivity to 73.4%, specificity to 94.8% and the chance of being normal (ppv-estimate) to 99.6% (95% CI: 98.8% - 100.0%), if > 3% of the spermatozoa expressed CYP24A1. (Figure 3 CD).

### 1,25(OH)₂D₃ increases intracellular calcium in human spermatozoa by a non-genomic action

By means of single cell calcium imaging using the calcium-sensitive probe fura-2-acetomethyl ester, we showed that 1,25(OH)₂D₃ (in 50 pM-10 µM concentration) induced a rapid increase in intracellular calcium in human spermatozoa (Figure 5A-C). The increase in intracellular calcium was 5-10 fold from baseline (40% of the amplitude elicited by 10 µM progesteron) and started in the neck region and propagated up in the post-acrosomal region and down through the midpiece of the spermatozoa (Figure 5A-C). It was an all or nothing response, which remained consistent and reproducible in the physiological range from 100 pM to 1 nM 1,25(OH)2D3 (Figure 5C).

The increase was initiated after a few seconds, and the steep increase was followed by a plateau phase before calcium levels slowly restore (Figure 5). The amplitude did not seem to increase markedly with higher concentration, but the duration of the plateau phase was longer. (Figure 5). The pattern resembled the effect elicited by progesterone, however, the amplitude was less and the effect on intracellular calcium of progesterone could not be abrogated by pre-treatment with 1,25(OH)₂D₃ (Figure 5).

By using a nuclear VDR antagonist ZK159222 and the non-genomic VDR antagonist 1β,25(OH)₂D₃ we showed that the effect of 1nM calcitriol could not be abrogated by ZK159222, but was completely blocked by pretreatment with 1-40 µM 1β,25(OH)₂D₃ (Figure 5). Unlike the effect of progesterone, which could not be blocked by any of the used VDR antagonists. Neither, 1,25(OH)₂D₃ nor progesterone were able to induce an increase in intracellular calcium in calcium free media (5 µM EGTA), despite that the spermatozoa were motile, low in intracellular calcium, and were only exposed shortly to low concentrations of EGTA.

To explore whether the intracellular stores were depleted by the addition of EGTA, we repeated the experiments and added after initiation-failure of 1,25(OH)₂D₃ and progesterone, 500 nM of ionomycin, which were able to induce a low but significant increase in the intracellular calcium in the calcium-free media.
To further evaluate the downstream mediators of the 1,25(OH)₂D₃ induced calcium release, we used the PLC inhibitor U73122 in normal calcium containing media. The majority of the human spermatozoa increased their baseline intracellular calcium concentration after addition of 5 µM U73122, however 10-20 % of the spermatozoa remained low and stable in intracellular calcium. The rapid increase in intracellular calcium elicited with 1 nM 1,25(OH)₂D₃ was delayed in these spermatozoa and the amplitude was smaller or absent after using 5µM U73122, indicating that PLC may be one of the downstream mediators of the activated VDR. We subsequently investigated whether 25-OH D₃ was able to elicit a response similar to calcitriol in the normal media. In spite of the difference in Kd we used 25-OH D₃ in concentrations up to 100nM, and observed no effect on intracellular calcium levels.

The immmunocytochemical results were supported by western blots (WB) (figure 10), which showed that VDR, CYP27B1, CYP2R1, CYP27A1 and CYP24A1 are expressed in semen samples from five men. We were not able to detect any substantial difference in the abundance of the proteins in the investigated semen samples. Although the amount of protein seemed higher after percoll separation, which indicates that good quality spermatozoa express the VD metabolizing machinery and there is a concomitant expression of all the metabolizing enzymes and the receptor.

### Example 2

### Introduction

Infertility in humans is estimated to affect 10-15% of couples and male fecundity is part of the problem in approximately 50% of all cases. Assessment of semen quality has a key role in the examination of male infertility, and the most predictive variables are total sperm count, sperm motility and percentage of morphologically normal spermatozoa.

Although valuable, assessment of semen variables has only limited predictive power, and several groups have attempted to develop alternative methods to assess male fertility potential. One approach is to investigate genes expressed late during spermatogenesis and explore whether these genes are important for sperm function, and subsequently determine if they affect the ability of spermatozoa to complete spermiogenesis, maturation in the epididymis and undergo capacitation and fertilize the egg.

The initial step towards an improved test of semen quality is to identify markers that are associated with the conventionally assessed semen variables and preferably provide additional information compared with standard semen examination.

Animal models indicate that impaired vitamin D (VD) signalling is associated with reduced male fertility, as both VD-deficient rats and VD receptor (VDR) knock out mice have impaired male fertility and a low number of motile spermatozoa. We have recently shown that VDR, and the VD metabolizing enzymes (CYP2R1, CYP27A1, CYP27B1, CYP24A1) are expressed in the human ejaculatory duct, in germ cells and in mature spermatozoa.

VDR and the VD-metabolizing enzymes are co-expressed late in spermatogenesis and in the neck and midpiece of a subpopulation of spermatozoa suggesting a role of VD in human reproduction (Blomberg Jensen, Nielsen et al., 2010). A role for VD in sperm function is supported by new functional human studies showing that VD through VDR activation increases intracellular calcium concentration, induces sperm motility and the acrosome reaction.

During our initial immunocytochemical investigations of VDR and the VD-metabolizing enzymes, we noticed that only few spermatozoa from infertile men, unlike normal men, expressed VDR and the VD metabolizing enzymes.

### Materials and methods

### Study subjects

We included male patients (subfertile men part of an infertile couple) from our andrology clinic and unselected young men from the general Danish population. All the young men were included during their compulsatory medical examination before determining suitability for military service, and their fertility potential was unknown due to their low age (normal men). All men had a physical examination performed, a blood sample drawn and delivered a semen sample. Spermatozoa from all men were investigated for CYP24A1 expression. To ensure that CYP24A1 expression was concomitantly expressed with VDR, we investigated spermatozoa from the first 10 men simultaneously using immunocytochemical (ICC) staining for VDR, CYP2R1 and CYP27B1 expression. Moreover, spermatozoa from 3 normal men were double stained for VDR and CYP24A1. The last 18 patients included in the series were also investigated for the annulus marker Septin 4 for comparison with CYP24A1 expression, while additional 69 men were investigated for VDR expression, 21 men for CYP27B1 expression and 9 men for CYP2R1 expression. Samples from 23 men were also used for analysis of CYP24A1 expression before and after percoll gradient centrifugation. Semen samples for the in vitro studies were obtained from 40 additional men (20 from the general population and 20 subfertile men). Afterwards, we extended the test by investigating CYP24A1 expression in 302 infertile men and 88 normal men.

### Immunocytochemistry (ICC) of spermatozoa

ICC was conducted after semen analyses, each sample was divided into aliquots of 100 µl following addition of 400 µl phosphate-buffered saline, before centrifuged (5 min, 300 x g) on a microscope slide ("cytospins") and dried without fixation. Antigen retrieval was accomplished by use of microwave for 15 min in TEG buffer. Then samples were washed in 0.5 % hydrogen peroxide followed by incubation with 2% non-immune goat serum (or BSA) to minimize cross-reactivity. Primary antibodies were purchased from Santa Cruz CYP2R1 (C-15) sc-48985, CYP27B1 (H-90) sc-67261, VDR (H-81) sc-9164, CYP24A1 (H-87) sc-66851, and Septin 4 (H-120) sc-20179. All antibodies were tested by western blot (WB) in human spermatozoa and VD related proteins were validated in human kidney samples by both IHC and WB . After 16 hours of incubation (VDR, 1:100, CYP27B1, 1:100, CYP24A1, 1:200 and Septin 4, 1:100) at 4°C, the cytospins were incubated with biotinylated goat anti-rabbit IgG, before a peroxidase-conjugated streptavidin complex was used as a tertiary layer. Visualization was performed with amino ethyl carbasole. Double ICC staining was performed according to the manufacturers manual (K3561 Envision G/2 rabbit/mouse Dako) except that treatment with CYP24A1 and VDR antibodies was for one hour at room temperature. Two independent investigators both blinded to patient data, evaluated all cytospins. Representative areas were selected (objective 40x) followed by examination using 100x objective magnification. The area was examined systematically and the percentage of spermatozoa expressing CYP24A1 at the annulus was calculated. The extended CYP24A1 expression study was conducted using duplicates and scored blinded to patient data.

### Semen analysis and reproductive hormones

All participants had been instructed to abstain from ejaculation 48 hours prior to delivery of their semen sample. Information of the actual time of abstinence was obtained. Assessment of semen samples was conducted routinely Briefly, weighing assessed semen volume; sperm motility was classified as progressive motile (WHO class A+B), non-progressive motile (class C) or immotile (class D). Sperm concentration was assessed using a Burker-Turk haemocytometer. Only spermatozoa with tails were counted. Smears were made, air dried, fixed in 96% ethanol and Papanicolaou stained for morphology analysis according to strict criteria. Serum FSH level was determined using a time-resolved immunofluorometric assay (Delfia; Wallac, Turku, Finland), and Inhibin-B by a specific two-sided enzyme linked immunoassay (Bio-Innovation Ltd, Oxford, UK) and meausurements of 25-hydroxy-vitamin D was conducted by isotope dilution liquid chromatography tandem mass spectrometry (LC-MS/MS) as described earlier.

### Semen analysis and reproductive hormones

The participants produced a semen sample by masturbation. Self-reported information of duration of ejaculation abstinence was obtained. Trained technicians conducted semen analysis Semen volume was estimated by weighing. For sperm motility assessment, duplicates of 10 µl of well-mixed semen were placed on a glass slide, examined on a heating stage kept at 37 °C, under a microscope at x 400 magnification, and spermatozoa were classified as progressive motile (WHO class A+B), non-progressive motile (class C) or immotile (class D). The average of the two motility assessments was used. For the assessment of the sperm concentration the samples were diluted in a solution of 0.6 mol/l NaHCO3 and 0.4% (v/v) formaldehyde in distilled water, and subsequently assessed using Burker-Turk haemocytometer. Only spermatozoa with tails were counted. Finally, smears were prepared, Papanicolaou stained and spermatozoa morphology assessed according to strict criteria .Serum FSH level was determined using a time-resolved immunofluorometric assay (Delfia; Wallac, Turku, Finland), and Inhibin-B by a specific two-sided enzyme linked immunoassay (Bio-Innovation Ltd, Oxford, UK).

### Semen samples for in vitro studies

Semen samples for the in vitro studies were obtained from 40 men from the general population, who were investigated between October 2009 and December 2010. Their semen samples were assessed as described above. To investigate the effect of VD on sperm motility, the semen samples were exposed to 1nM 1,25(OH)2D3 for 45 minutes and motility scored as described earlier. Motility assessment was conducted in 17 samples (in duplicates) under non-capacitating conditions (in their seminal plasma), and 3 samples were also analyzed in capacitating conditions. 7 consecutively included men were used to investigate a dose-response relationship between VD and sperm motility.
Calcium measurements and the acrosome reaction were performed on motile spermatozoa isolated after percoll density gradient centrifugation (Supra sperm gradients, (ORIGO)) following the manufacturer's instructions. Briefly, spermatozoa were separated from seminal plasma by centrifugation on a discontinuous percoll gradient using a two-step gradient comprising 2 ml layers of 55% and 80% percoll respectively. Semen was placed on the top of the gradient and centrifuged at 300 x g for 25 min. The spermatozoa at the base of the 80% fraction were collected and washed with a 5 ml volume of capacitating medium (CaCl2 1,5mM, KCl 4.3 mM, NaHCO3 31mM, HEPES 15mM, Human Serum Albumin 5mg/ml, Glucose 4.5mM, Sodium Pyruvate 0.8mM, Synthetic Serum Replacement, 0.5 ug/ml human recombinant insulin (Sperm preparation media (ORIGO)) and finally resuspended in capacitating media and used for the following assays of calcium measurements and acrosome reaction. All men were part of the above mentioned ongoing surveillance program of semen quality among young Danish men and included after approval from the local ethical committee.

### Effect of VD on calcium levels in human spermatozoa

Semen samples were separated by percoll density gradient centrifugation to ensure viable and motile spermatozoa and resuspended in capacitating media for 1.5 hours. Afterwards, loaded with 3 µM of the fluorescent probe (intracellular calcium binding) fura-2/AM (Invitrogen, USA) for 30 min, centrifuged (10 min at 300 g) and washed with Krebs-Ringer buffer (120 mM NaCl, 15 mM NaHCO3, 5 mM KCl, 1 mM CaCl2, 5 mM Na2HPO4, 1 mM MgCl2 pH 7.3). In a Ca-free Krebs Ringer solution CaCl2 was substituted for 5 mM EGTA (calcium free with EGTA). Subsequently, 80 µl of the sperm sample was diluted in 920 µl Krebs-Ringer buffer and loaded on polylysine-coated chambers to minimize movement of the spermatozoa (Lab-Tek; Nalge Nunc International, USA). A Zeiss Axiovert 135 microscope equipped with a Zeiss Achrostigmat 40x 1.3 NA objective was used to acquire images from the fluorescent probe. Excitation was obtained by a Polychrome Villuminator from Till Photonics (Germany) and images were acquired using a Cool Snap CCD camera (Photometrics, USA) from Robert Scientific (Malaysia). For measurements of [Ca2+]i, the excitation wavelengths were 338 and 380 nm, measuring emission above 510 nm using a cut-off filter. Calculations of [Ca2+]i were conducted by MetaFluor software from Molecular Devices and using a Kd of 160nM . After background subtraction, ratio images were formed using MetaFluor software. Chemicals, 1α,25(OH)2D3 (1,25(OH)2D3), 25-OH D3, progesterone, thapsigargin, nifedipine and ionomycin were purchased from Sigma-Aldrich (USA), PL were carried out in triplicates C-inhibitor U73122 (1-[6-[[17fi-3-methoxyestra-1,3,5(10)-trien-17-yl]amino]hexyl]-1H-pyrrole-2,5-dione) from Biomol, PA, U.S.A. The non-genomic VDR antagonist 1β,25-(OH)2D3 was a gift from LEO Pharma (Denmark).

All experiments started by using low magnification to ensure visibility of several spermatozoa simultaneously. When a color change was observed in most cells, higher magnification was used. All measurements of [Ca2+]i were based upon evaluation of at least three spermatozoa simultaneously (see exact number in figure legends), and were repeated in at least three separate experiments. We used 10 different semen donors to ensure that all experiments were reproduced on semen samples from at least two different men.

### Acrosome reaction

Acrosome status was assessed according to a modified version of the method described by Cross et al. 1986. Motile spermatozoa were isolated by percoll gradient centrifugation, washed twice with 5 ml 0.9% sodium chloride followed by centrifugation at 300 x g for 10 min. Hereafter, the motile spermatozoa were resuspended with or without 1 nM 1,25(OH)2D3 for 1.5 hours at room temperature in 0.9% sodium chloride or resuspended in capacitating media for 1.5 hours at 37° C and 5% CO2 before the sperm was smeared on a glass slide and a cytospin was created. The smear and cytospin were fixed in 96% ethanol for 5 min after air drying, washed in distilled water and stained for minimum 2 hours with Pisum sativum agglutinin labelled with fluorescein isothiocyanate (Sigma Aldrich) in Dulbecco phosphate buffered saline at 4°C. Finally, the slides were washed in distilled water and mounted with Vectrashield (H-1080 Vectar Laboratories) and 200 spermatozoa were counted on each slide using a fluorescence microscope at a magnification of 400x. The mean percentage of acrosome-reacted spermatozoa of the two slides was calculated. When more than half the head of a spermatozoon was brightly and uniformly fluorescent, the acrosome was considered to be intact. Spermatozoa without fluorescence or with a fluorescing band limited to the equatorial segment were considered to be acrosome-reacted. Spermatozoa with a fluorescing band limited to the equatorial segment were named eq-AR. The person assessing the slides was blinded to the treatment of the sample.

### Statistics

Differences in basic characteristics between subfertile and normal men were tested by ANOVA, and correlations between CYP24A1 expression and semen variables were tested by Spearman's correlation. Between group differences between semen variables and CYP24A1 expression were tested by regression analyses. A Receiver Operating Characteristics (ROC) curve was used to estimate the diagnostic properties of CYP24A1 as a marker of semen quality. Positive predictive values (ppv) and odds ratio were calculated. The results from induction of motility were tested using paired t-test (two-tailed). For all analyses p<0.05 was considered statistically significant. Statistical analyses were performed using PASW software version 18 and SAS version 6.1.

### Results

### Expression of CYP2R1, CYP27B1, VDR and CYP24A1 in human spermatozoa

Fig. 12 illustrates expression of CYP2R1, CYP27B1, VDR and CYP24A1 at neck of human spermatozoa from young, normal men. All proteins were also expressed in other subcellular locations.

The predominant location for VDR and CYP27B1 was in the neck and post-acrosomal region, while CYP2R1 was mainly expressed at the tail. CYP24A1 was primarily expressed at the annulus of normal spermatozoa, although it was frequently detected in the neck with concomitant expression at the annulus. Sperm with no detectable CYP24A1 expression at the annulus were in most cases (approx. 80%) not expressing CYP24A1 in other subcellular locations. Septin 4 was solely detected at the annulus (Fig. 2 and 12) and expression was found in a higher proportion of spermatozoa compared to CYP24A1 expression at the annulus [median/mean]: Septin [4%/26%] and CYP24A1, [2%/5%], p<0.01 (Fig. 2). Double staining of VDR and CYP24A1 revealed that approximately 80 % of the spermatozoa from normal men had concomitant expression of both proteins and were thus either expressing both proteins or none of them on each spermatozoa (Fig. 2).

Evaluation of CYP24A1 expression by two blinded investigators showed high agreement. Only 8% of the slides were scored markedly different (more than 15% difference, or one of the investigators scored below the threshold set at >3% (see the following paragraph) and the other above). In such cases the slides were reevaluated simultaneously by both investigators.

### VDR, CYP2R1 and CYP27B1 expression in spermatozoa from young men from the general population and subfertile men

Fig.13 shows the mean expression of VDR in spermatozoa from normal and infertile men. VDR expression is significantly higher (p=0.003) in spermatozoa from normal men compared with infertile men. The same pattern of expression was found for CYP2R1 with a mean expression of 53% in normal and 27% in the infertile men, while the difference was modest for CYP27B1 with a mean of 15% and 13% respectively.

### CYP24A1 expression in spermatozoa from young men from the general population and subfertile men

Fig. 1 and 12 illustrate presence of CYP24A1 at the annulus and neck of human spermatozoa from a young, normal man with good semen quality and absence in spermatozoa from a subfertile man. The young normal men had a higher percentage of spermatozoa expressing CYP24A1 at the annulus compared with subfertile men (p<0.0005) (Fig. 3 and 17). They also had a higher total sperm count, sperm concentration, percentage of motile and morphologically normal spermatozoa (p<0.0005) compared to subfertile men (Figure 7).
A ROC curve (data not shown) indicated that there seemed to be a threshold at >3% CYP24A1-positive annuli, and 59 of the 77 subfertile men had ≤3% spermatozoa expressing CYP24A1 . These 59 subfertile men had significantly lower total sperm number compared with the 18 having >3% of the spermatozoa expressing CYP24A1 . Basic characteristics of these 18 subfertile men (named normospermic) are given .in Figure 8. Among these, > 80% had a total sperm count ≥ 40 x 106 spermatozoa, ABC-motility ≥ 40%, FSH < 8 IU/I and Inhibin B> 50 pg/ml. Sixteen of these men were partners of an infertile couple with clearly defined female infertility factors, 6 had previously fathered a child or been responsible for a pregnancy, and few were part of a couple with unexplained infertility.

Sperm morphology varied markedly in the group of subfertile men (0.0-13.5%) and we found no difference in morphology between the two subgroups of subfertile men. The threshold set at >3% CYP24A1-positive spermatozoa to distinguish normal men from the general population from subfertile men resulted in a sensitivity of 67.9%, specificity 70.2% and chance of being normal (ppv-estimate) of 97.7% [96.3% - 98.7%], while the odds ratio (OR) for being normal was 5.0 [2.3-10.5]. If the normospermic partners of infertile couples were removed, the sensitivity increased to 73.4%, specificity 94.8% and ppv-estimate 99.6% (98.8% - 100.0%). 17 young normal men had an expression of CYP24A1 below the >3% threshold of CYP24A1-positive spermatozoa. They did not differ significantly from the remaining men from the general population, although they had a lower mean sperm concentration and a lower percentage of motile and morphologically normal spermatozoa.

We have now extended this study by investigating additional 380 men (300 infertile and 80 normal men), in which the mean expressing of CYP24A1 in normal men was 36,1% compared with 1.4% in the infertile men.

### CYP24A1 expression and semen quality

CYP24A1 expression at the annulus was positively correlated with total sperm count (r= 0.50, p<0.0005), sperm concentration (r=0.52, p<0.0005), percentage of motile sperm (r=0.33, p<0.0005), progressive motile sperm (r=0.25, p=0.01), and morphologically normal spermatozoa (r=0.26, p<0.005) (Figure 4A-E). All men were stratified in four different groups according to total sperm number, - concentration, -motility, progressive motility and morphology. The mean CYP24A1 expression increased from the lowest to the highest group for all variables, although some groups included less than 15 men, which resulted in large confidence intervals. Using regression analyses we found significant differences comparing the highest group (highest quality for each variable) with the lower groups for all the semen variables (all p<0.01). The percentage of CYP24A1 positive spermatozoa increased in 17 out of 23 patients after percoll density gradient centrifugation (median 15 % versus 41 %, p<0.0005), and in the remaining 6 men the percentage were unchanged or with a minor decline (Figure 4).

### Vitamin D and intracellular calcium in human spermatozoa

Addition of 1,25(OH)2D3 (100 pM-10 µM) to the motile spermatozoa induced a rapid increase in intracellular calcium ([Ca2+]i) (Figure 5 and 14). The increase was 5-10 fold from baseline calcium level and started within 5 seconds in the neck region and propagated up in the post-acrosomal region and down through the proximal midpiece (Figure 5). This effect was reproducible from 100 pM, and the threshold for the observed all-or-nothing response was between 10-50 pM 1,25(OH)2D3 (Figure 5). The steep increase in [Ca2+]i was followed by a short plateau phase (up to 50 sec for < 10 nM 1,25(OH)2D3 ) until [Ca2+]i restored back to baseline concentration (Figure 5).

The amplitude did not increase with higher 1,25(OH)2D3 concentrations, but the duration of the plateau phase was longer (50 -300 sec). The maximum amplitude (max amplitude) of the calcium increase induced by 1 nM 1,25(OH)2D3 varied between the investigated men. It was 5-10 fold higher than baseline calcium level and approximately 40-80 % of the max amplitude elicited by 10 µM progesterone (Supplementary figure 2). The effect of progesterone could not be abrogated by pre-treatment with 1,25(OH)2D3 (Figure 5). The nuclear VDR antagonist (ZK159222, 0.1-100 µM) was unable to abrogate or suppress the effect of 1,25(OH)2D3 treatment (data not shown), but the VD response was blocked completely by pretreatment with 1-40 µM of the non-genomic VDR antagonist 1β,25(OH) 2D3 (Figure 5).

Pretreatment with nifedipine 10 µM did not affect the response to 1,25(OH)2D3. The calcium increase was initiated rapidly with a maximum amplitude 7 fold higher than baseline, which was followed by 4 minutes long repolarisation phase. However, nifedipine treatment caused an aberrant response to progesterone. The rapid initiation and steep increase in calcium seemed unaffected, but the plateau phase was terminated rapidly by a slow repolarisation phase, and the calcium level was restored to baseline level 9 minutes after initiation of progesterone treatment .Treatment with thapsigargin 4 µM caused a slow increase in baseline calcium concentration in all spermatozoa .Nine minutes after thapsigargin exposure it reached a stable plateau 3 fold higher than baseline calcium concentration. Addition of 1,25(OH)2D3 (9 min after initiation of thapsigargin treatment) to these spermatozoa was unable to elicit a substantial calcium response, while lower concentrations of thapsigargin (< 1 µM) were unable to abrogate the effect of 1,25(OH)2D3.

To test the mechanism downstream to the VDR, we used a PLC inhibitor U73122, to investigate whether VDR signalling was mediated by a PLC-dependent IP3 production, which may induce an intracellular calcium release. After exposure to 10 µM U73122, the majority (> 80%) of the spermatozoa increased their baseline [Ca2+]I more than 5 fold, most were immotile and were unresponsive to treatment with VD. To avoid bias from dying spermatozoa, we changed the concentration of U73122 to 2 µM, where most of the spermatozoa remained low and did not fluctuate in [Ca2+]I and were motile. 1,25(OH)2D3 was unable to induce a rapid increase in [Ca2+]i in spermatozoa exposed to 2 µM U73122. The increase in calcium was instead very slow with long lasting (several minutes) increase unlike the normal rapid response, where the max amplitude was achieved within one minute. The maximum amplitude was also lower, while the plateau phase was longer caused by a delayed repolarisation . PLC inhibition was unable to abrogate the effect of progesterone (data not shown).

We observed a marked response to 1,25(OH)2D3 in a calcium free media (potentially contaminated [Ca2+] < 5 uM) without EGTA . The response to 1,25(OH)2D3 was aberrant in this media. Here, we observed a longer plateau phase and a delayed repolarisation phase compared to the normal calcium containing media. However, 1,25(OH)2D3 or progesterone could not induce an increase in [Ca2+]i in calcium free media with 5 µM EGTA, despite the spermatozoa being motile, low in baseline [Ca2+]i and exposed shortly (< 20 min) to low concentrations of EGTA .Ionomycin (500 nM) induced a low but reproducible increase in [Ca2+]i in the calcium free media with EGTA . The amplitude of the response to ionomycin was low (< 3 fold from baseline), indicating that the intracellular Ca2+ stores could be partially emptied. Addition of 1 mM Calcium to the calcium free media after treatment with either 1,25(OH)2D3 or progesterone resulted in an increase in [Ca2+]I, which was comparable to the increase observed in the normal calcium containing Krebs Ringer solution although the plateau phase was longer in the spermatozoa exposed initially to a calcium free media . 25-OH D3 (100nM) was unable to elicit an increase in [Ca2+]i. Resuspending the motile spermatozoa in non-capacitating media resulted in a lower amplitude to 1 nM 1,25(OH)2D3 treatment, but a comparable response pattern to the effects observed in capacitating media . We noticed that some of the spermatozoa did not respond to the addition of 1,25(OH)2D3 by increasing [Ca2+]i, but remained low in [Ca2+]i. These VD-unresponsive cells (marked with arrowhead in Fig. 14 rapidly increased in [Ca2+]i after progesterone stimulation and were thus viable cells (Fig. 14).

### Vitamin D induces sperm motility and the acrosome reaction in vitro

We investigated a dose-response relationship between 1,25(OH)2D3 and the induction sperm motility (ABC motility). We tested the effect of 1,25(OH)2D3 in the concentration range from 10-15M to 10-3M and found a non-linear dose-response relationship. We found a modest increase in median sperm motility between 2-7 % in the concentration range from 10-14M to 10-9M . The concentration range from 10-12 to 10-9M induced the highest increase in motility, and the strongest induction was found for 10-9M with a median increase of 7% (p=0.006). Concentrations above 10-7M resulted in a decreased motility compared with control (10-5M, median 15% decrease). We extended this by studying semen samples from 17 men with and without exposure to 10-9M 1,25(OH)2D3 for 30 min at room temperature between 1-3 hours after ejaculation. Their mean sperm motility increased significantly (p= 0.010) after VD exposure with 2.9%, 95% CI [1%;5%]. Most men had a modest increase in sperm motility although a subset (n=3) of the men had a slight decrease in motility (Figure 14). We have previously shown that sperm motility (ABC) increased following treatment with 1 nM 1,25(OH)2D3. The mean sperm motility increased significantly (p= 0.01) in the young normal men after VD exposure, while sperm motility did not increase (p=0.80) following 1,25(OH)2D3 treatment in the infertile men (Figure 14).

1,25(OH)2D3 (10-9M) significantly (p=0.024) increased the number of acrosome reacted spermatozoa with an average increase of 6%, 95CI [1%;10%] ( median, control group 12% and VD group 21 %), while the percentage of spermatozoa with an equatorial band (eq.AR) increased from a median of 4.7% to 5.3% (p=0.214) (Figure 15). 6 men were investigated in non-capacitating conditions (NaCl), and there was no significant difference between VD treated and the control group (4.2% versus 3.7%).

### Discussion

In this study of infertile men and young men from the general Danish population, we show that assessment of CYP24A1 expression at the annulus or expression of VDR, CYP27B1, CYP2R1 of human spermatozoa posses the potential to be used as an objective proxy or a valuable supplement to routine semen analysis. Several other markers have been suggested as suitable markers for screening of male fertility potential, but none of these are used routinely in a clinical setting.

A threshold of >3% CYP24A1 positive spermatozoa resulted in a relatively high specificity and an OR of 5 for being a normal man. Moreover, test results above this threshold indicated a high chance of having high total sperm number, many motile and morphologically normal spermatozoa, which was supported by the strong positive correlations between CYP24A1 expression and semen quality variables. A limitation of the interpretation of our results is the unknown fertility potential of our control group (young men from the general population) due to their young age. Thus, validation in an independent cohort with known fertility status is mandatory before the test can be applied in a clinical setting. However, we believe that CYP24A1 expression is a promising candidate as a marker for semen quality and there is a need for an objective marker with high reproducibility as an addition to routine semen analysis. Presence of CYP24A1 at the annulus of the spermatozoa seems to be the discriminating factor for sperm quality, rather than being solely an annulus marker, because CYP24A1 expression was found in fewer spermatozoa than the annulus marker Septin 4.

One or both partners in combination can be responsible for infertility. Around 20% of all infertility cases are attributable solely to the male partner, a subfertile male contributes to the problem in further 25%, and in ~ 15% no factor can be defined in either partner. Noteworthy, low or absent CYP24A1 expression was observed mainly among patients in whom conventional parameters of semen quality were impaired. Furthermore, men from infertile couples, who had >3% of spermatozoa expressing CYP24A1 were classified as normospermic men from infertile couples, because most of these men had normal sperm counts with good motility and normal levels of Inhibin B and FSH.

However, the normospermic subfertile men had a low proportion of morphologically normal spermatozoa, and sperm morphology is known to be an important predictor of fecundity. This could be a weakness of this marker, because previous studies have associated normal sperm morphology below 10-12% with impaired fertility. However, CYP24A1 expression was strongly positively correlated with sperm morphology, but the mean number of morphologically normal spermatozoa was only 4 % in this subgroup and thus substantially lower than the normal men.

On the contrary, progressive sperm motility and sperm motility are some of the best predictors of male fertility potential, and CYP24A1 were strongly correlated with both of these motility variables.

This was further supported by the high increase in CYP24A1 expression in the progressive motile spermatozoa isolated after percoll gradient centrifugation. This indicates that presence of the VD metabolizing machinery is associated with good semen quality, which is in line with results from both VDR knock mice and rat VD deficiency models that both results in impaired fertility and few motile spermatozoa.

We also identified normal men with apparently normal semen quality, who had a low proportion of spermatozoa (≤3%) expressing CYP24A1. Unfortunately, we are unaware of the normal control's fertility potential that may influence this finding, but in some cases the results were caused by methodological difficulties (substandard staining). However, a few samples analyzed simultaneously for the annulus marker Septin 4 showed that the expression of Septin 4 was also low, indicating that they could have a failure in annulus formation and potentially impaired fertility.

Male infertility is a heterogeneous disorder. In most cases, the pathogenesis involves impairment of either sperm production, maturation or function. Generally, more than 80% of human spermatozoa are abnormal and only few posses the ability to swim upwards in the female reproductive tract, undergo capacitation, bind to and subsequently fertilize the egg. It is likely that absence or low expression of CYP24A1 could be a secondary event caused by testicular dysfunction; and the low protein expression could be the result of impaired spermatogenesis, incomplete maturation or other defects resulting in the formation of abnormal spermatozoa. Alternatively, VD may be important for sperm quality and reproductive function as has been suggested by both animal and human studies.

VD is important for calcium homeostasis and VD synthesis normally starts in the skin with UV-B conversion of 7-dehydrocholesterol to inactive VD. Then, follows a two-step enzymatic activation in the liver (CYP2R1) and kidney (CYP27B1), before the active 1,25(OH)2D3 binds and activates VDR in the target tissue until inactivated by CYP24A1 (Prosser and Jones, 2004). Local presence of VDR and VD-metabolizing enzymes has been implicated in several cellular functions besides calcium homeostasis and seems to be regulated differently than the systemic metabolism.

The main determinant of the local VD response is the cellular abundance of 1,25(OH)2D3, and since 1,25(OH)2D3 is a strong inducer of CYP24A1 transcription though vitamin D response elements in the promotor, we speculate that the presence of CYP24A1 in human spermatozoa could be the result of substantial VD exposure during spermatogenesis.

As we found no association between VD serum levels and CYP24A1 expression in spermatozoa, we hypothesize that the presence of VD-metabolizing machinery in testis and spermatozoa is likely to be dependent on other paracrine regulators besides the circulating serum level of 25-hydroxy-VD. This implies that the presence of VDR and the VD metabolizing enzymes may be merely dependent on optimal differentiation and maturation of human spermatozoa than solely the serum level of VD progenitors, and we suggest that the presence of CYP24A1 in mature spermatozoa may function as a gatekeeper to calibrate the action of VD in human spermatozoa during epididymal transit or in the female reproductive tract. VD has been suggested to induce motility, survival, capacitation, and the acrosome reaction in human spermatozoa.

VDR and the VD metabolizing enzymes are concomitantly expressed, and the low CYP24A1 expression observed in spermatozoa from infertile men suggested that the expression of VDR and the VD activating enzymes also may be low in these spermatozoa.

This hypothesis was supported by the double staining experiments, which showed concomitant expression of VDR and CYP24A1 in the same spermatozoa and by showing that expression of VDR, CYP2R1 and CYP27B1 was able to distinguish spermatozoa from normal and infertile men. Although, the test sample was smaller for these proteins resulting in lower specificity than CYP24A1. This implies that a proportion of spermatozoa posses the VD metabolizing machinery and responds to 1,25(OH)2D3, while the remaining spermatozoa with no VDR expression are unresponsive. Presence of VDR and the VD metabolizing enzymes in the neck of mature spermatozoa indicates that VD may have a function in the female reproductive tract. The concentration of VD progenitors in both female and male reproductive tract has to our knowledge not been shown, although the concentration of 1,25(OH)2D3 in the follicular fluid during in vitro fertilization has been measured and suggested to be cyclic with the highest concentration at ovulation.

VD is metabolized locally in the male reproductive tract, and the concentrations of 1,25(OH)2D3 that induced an effect in the in vitro studies are close to the physiological concentrations in serum (≈ 1 x 10-10M), other somatic tissues, and to the dissociation constant for VDR ≈ 1-7 x 10-10M. The rapid increase in intracellular calcium was initiated in the neck of the spermatozoa, where VDR co-localize with the redundant nuclear envelope (RNE). RNE is thought to be a calcium storage, and the Ca2+ release following 1,25(OH)2D3 treatment was rapid; initiated within seconds and is presumably a non-genomic effect. Spermatozoa are transcriptionally silent and a non-genomic effect of 1,25(OH)2D3 was confirmed by using the non-genomic VDR antagonist 1β,25(OH)2D3, which abrogated the response to 1,25(OH)2D3 completely.

The effects downstream to the VDR were elucidated by showing that nifedipine was unable to affect the VD response, unlike the progesterone response, in which the plateau phase was terminated rapidly by inhibition of the voltage gated L-type calcium channel in accordance with an earlier publication. VD-mediated calcium release from an intracellular calcium storage was supported by showing that thapsigargin diminished the effect of 1,25(OH)2D3, although there exist some controversy regarding the concentrations needed to block the SERCA pumps in human spermatozoa. It has been demonstrated by double staining that the calcium binding protein calreticulin reside in the neck of human spermatozoa where it co-localize with the 1,4,5-trisphosphate receptor (IP3R), and this putative calcium storage has been suggested to be important during capacitation.

We have previously shown that the VDR and all the VD metabolizing enzymes are expressed at the neck of human spermatozoa, and since 1,25(OH)2D3 is a known non-genomic inducer of PLC in other mesenchymal derived tissues, we investigated whether PLC inhibition was able to abrogate the calcium release induced by 1,25(OH)2D3.

The PLC-inhibitor U73122 caused a delayed and diminished response to 1,25(OH)2D3 indicating that activated VDR may signal partly through PLC activation and hence increase IP3, which through activation of IP3R gated calcium channels in RNE releases calcium from an intracellular storage.

To validate the calcium release from intracellular stores, we investigated the response to VD and progesterone in calcium free media without EGTA. 1,25(OH)2D3 increased intracellular calcium rapidly, indicating that the calcium increase comes from intracellular stores. We also noticed that the phase of repolarization was aberrant resulting in a extended plateau phase, and we speculate that spermatozoa may have a calcium sensing receptor or be sensitive to changes in calcium homeostasis since the same phenomenon was observed after addition of 1mM calcium to EGTA containing calcium free media and in the experiment with the PLC-inhibitor. When using a Ca-free Krebs Ringer solution with a low EGTA concentration, we noticed that the effect of both 1,25(OH)2D3 and progesterone was abrogated, and ionomycin elicited only a small Ca2+ rise when added to the solution. It has previously been suggested that the IP3 gated calcium storage in the RNE can be rapidly depleted by EGTA. This may explain the low amplitude induced by ionomycin in the calcium free media, which ultimately could be caused by calcium release from other compartments, such as mitochondria.

The VD-induced calcium increase in human spermatozoa indicates that VD may be involved in the induction of motility, and 1,25(OH)2D3 was able to induce a modest increase in sperm motility, which is in accordance with a previous study showing that VD increased calcium on sperm lysates, induced motility, and increased acrosin activity.

We found that 1,25(OH)2D3 induced a significant higher proportion of motile spermatozoa to undergo the acrosome reaction (under capacitating conditions), which may be caused by propagation of the calcium increase from the neck to the head of the spermatozoa.

During this investigation, we noticed that few of the progressive motile spermatozoa isolated from normal men after percoll gradient centrifugation were unresponsive to 1,25(OH)2D3 treatment. The lack of response to VD treatment in these few cells could be influenced by cell viability. However, it seemed not to be the case, as the spermatozoa remained responsive to progesterone treatment. This implies that a minority of high quality spermatozoa may not express VDR and are therefore unresponsive to 1,25(OH)2D3. Presence of VDR in the majority of the spermatozoa from young normal men was evident since 1,25(OH)2D3 was able to induce a modest increase in sperm motility. Unlike in the subfertile men, in which, only a subset were responsive to 1,25(OH)2D3 treatment resulting in no increase in sperm motility. This suggests that VDR may be expressed in fewer spermatozoa from infertile men, and that the low VDR expression may have functional consequences a these cells are unresponsive to 1,25(OH)2D3, which ultimately could aggravate their semen quality or their fertility chances, if VD signalling is important in the female reproductive tract.

In conclusion, this investigation can be considered a baseline study suggesting that expression of VDR and the VD metabolizing enzymes may serve as markers for semen quality. Further studies have validated that especially CYP24A1 is a strong predictive marker of semen quality, although its predictive value in an independent cohort needs to be determined and to determine if the expression can be used as a proxy for semen analysis, or whether it adds to the knowledge obtained by routine semen analysis and thus be a valuable addition for evaluation of male fertility potential.

### References

Aquila S, Guido C, Perrotta I, Tripepi S, Nastro A, Andò S. Human sperm anatomy: ultrastructural localization of 1alpha,25-dihydroxyvitamin D receptor and its possible role in the human male gamete. J Anat. 2008 Nov;213(5):555-64.
Aquila S, Guido C, Middea E, Perrotta I, Bruno R, Pellegrino M and Andò S. Human male gamete endocrinology:1aplhpa,25-dihydroxyvitamin D3(1,25(OH)2D3) regulates different aspects of human sperm biology and metabolism. Reprod Biol Endocrinol.2009;7:140.
Blomberg Jensen M, Nielsen JE, Jørgensen A, Rajpert-De Meyts E, Kristensen DM, Jørgensen N, Skakkebaek NE, Juul A, Leffers H. Vitamin D receptor and vitamin D metabolizing enzymes are expressed in the human male reproductive tract. Hum Reprod. 2010 May;25(5):1303-11. Epub 2010 Feb 18. PMID: 20172873
Blomberg Jensen M, Andersen CB, Nielsen JE, Bagi P, Jørgensen A, Juul A, Leffers H. Expression of the vitamin D receptor, 25-hydroxylases, 1alpha-hydroxylase and 24-hydroxylase in the human kidney and renal clear cell cancer. J Steroid Biochem Mol Biol. 2010 Apr 1. PMID: 20362668
Kinuta K, Tanaka H, Moriwake T, Aya K, Kato S, Seino Y. Vitamin D is an important factor in estrogen biosynthesis of both female and male gonads. Endocrinology. 2000 Apr;141(4):1317-24.
Nagakura K, Abe E, Suda T, Hayakawa M, Nakamura H, Tazaki H. Inhibitory effect of 1 alpha,25-dihydroxyvitamin D3 on the growth of the renal carcinoma cell line. Kidney Int. 1986 Apr;29(4):834-40.
Panda DK, Miao D, Tremblay ML, Sirois J, Farookhi R, Hendy GN, Goltzman D. Targeted ablation of the 25-hydroxyvitamin D 1alpha -hydroxylase enzyme: evidence for skeletal, reproductive, and immune dysfunction. Proc Natl Acad Sci U S A. 2001 Jun 19;98(13):7498-503.
Prosser D. E. and Jones G. Enzymes involved in the activation and inactivation of vitamin D. Trends Biochem. Sci. 2004 29, 664-673.

## Claims

1. A method for predicting the fertility potential of a male mammal, said method comprising the steps of;
a) in a semen sample provided from said male mammal,
b) determining the expression level of CYP24A1,
c) selecting a desired sensitivity and specificity for a reference level,
d) determining that said male mammal is likely to have a normal fertility potential, if said determined expression level is equal to or above the reference level and/or determining that said mammal is unlikely to have a normal fertility potential, if said determined expression level is below the reference level.

2. A method according to claim 1, wherein the expression of the protein is detected at least at one of the locations on the sperm cells selected from the group consisting of the post-acrosomal region, neck, midpiece and the annulus.

3. A method according to claim 2, wherein the expression of the protein is determined at the annulus.

4. A method according to any of claims 1-3, wherein said expression level is determined by measuring the level of mRNA and/or protein.

5. A method according to claim 4, wherein said determination of the at least one protein level is performed using a method selected from the group consisting of immunohistochemistry, immunocytochemistry, FACS, ImageStream, Western Blotting, qPCR, RT-PCR, qRT-PCR, ELISA, Luminex, Multiplex, Immunoblotting, TRF-assaýs, immunochromatographic lateral flow assays, Enzyme Multiplied Immunoassay Techniques, RAST test, Radioimmunoassays, immunofluorescence and immunological dry stick assays.

6. A method according to claim 5, wherein said determination is performed by immunocytochemistry.

7. A method according to claim 5, wherein said determination is performed by ELISA.

8. A method according to any of the preceding claims, wherein the expression level is combined with expression level of at least one protein selected from the group consisting of Septin 4, Septin 12, Protamin 1, Protamin 2, FGFR1OP1, FGFR1OP2, FGFR1, Calbindin, TRPV6.

9. Use of CYP24A1 as a marker for male fertility potential in a male mammalian semen sample, wherein said male mammal is likely to have a normal fertility potential, if the level of CYP24A1 is equal to or above a reference level and/or determining that said mammal is unlikely to have a normal fertility potential, if the level of CYP24A1 is below the reference level.

10. A method for predicting the fertility potential of a male mammal, said method comprising the steps of;
a) in a semen sample provided from a male mammal,
b) adding some phosphate buffered saline to the sample,
c) spinning down the sample on a slide,
d) optionally incubating the slide at 4 °C for 1-1000 days,
e) detecting the expression of CYP24A1 in said sample on said slide, and
f) determining that said male mammal is likely to have a normal fertility potential if said determined expression level is equal to or above a reference level and/or determining that said mammal is unlikely to have a normal fertility potential if said determined expression level is below the reference level.

## Patentansprüche

1. Verfahren zur Vorhersage des Fertililtätspotenzials eines männlichen Säugetiers, wobei das Verfahren folgende Schritte umfasst;
a) in einer von dem männlichen Säugetier bereitgestellten Samenprobe
b) Ermitteln des Expressionsniveaus von CYP24A1,
c) Auswählen einer gewünschten Sensitivität und Spezifität eines Vergleichsniveaus,
d) Bestimmen, dass das männliche Säugetier wahrscheinlich ein normales Fertilitätspotenzial aufweist, wenn das bestimmte Expressionsniveau mindestens gleich dem Vergleichsniveau ist, und/oder Bestimmen, dass das männliche Säugetier wahrscheinlich kein normales Fertilitätspotenzial aufweist, wenn das bestimmte Expressionsniveau unter dem Vergleichsniveau ist.

2. Verfahren nach Anspruch 1, wobei die Expression des Proteins an mindestens einem der Bereiche der Samenzellen, ausgewählt aus der Gruppe, bestehend aus der postakrosomalen Region, Hals, Mittelstück und Annulus, nachgewiesen wird.

3. Verfahren nach Anspruch 2, wobei die Expression des Proteins am Annulus bestimmt wird.

4. Verfahren nach einem der Anspruch 1-3, wobei das Expressionsniveau durch Messen des mRNA- und/oder Proteingehalts bestimmt wird.

5. Verfahren nach Anspruch 4, wobei die Bestimmung des mindestens einen Proteingehalts mit einem Verfahren durchgeführt wird, ausgewählt aus der Gruppe, bestehend aus Immunohistochemie, Immunozytochemie, FACS, ImageStream, Western Blotting, qPCR, RT-PCR, qRT-PCR, ELISA, Luminex, Multiplex, Immunoblotting, TRF-Assays, immunchromatografische Lateralfluss-Assays, EMIT (Enzyme Multiplied Immunoassay Techniques), RAST-Test, Radioimmunoassays, Immunfluoreszenz und Immunoassays mit trockenen Teststreifen.

6. Verfahren nach Anspruch 5, wobei die Bestimmung mittels Immunozytochemie durchgeführt wird.

7. Verfahren nach Anspruch 5, wobei die Bestimmung mittels ELISA durchgeführt wird.

8. Verfahren nach einem der vorhergehenden Ansprüche, wobei das Expressionsniveau mit dem Expressionsniveau von mindestens einem Protein kombiniert wird, ausgewählt aus der Gruppe, bestehend aus Septin 4, Septin 12, Protamin 1, Protamin 2, FGFR1OP1, FGFR1OP2, FGFR1, Calbindin, TRPV6.

9. Verwendung von CYP24A1 als Marker für das männliche Fertilitätspotenzial einer Samenproben eines männlichen Säugetiers, wobei das männliche Säugetier wahrscheinlich ein normales Fertilitätspotenzial aufweist, wenn das CYP24A1-Niveau mindestens gleich einem Vergleichsniveau ist, und/oder Bestimmen, dass das männliche Säugetier wahrscheinlich kein normales Fertilitätspotenzial aufweist, wenn das CYP24A1-Niveau unter dem Vergleichsniveau ist.

10. Verfahren zur Vorhersage des Fertililtätspotenzials eines männlichen Säugetiers, wobei das Verfahren folgende Schritte umfasst;
a) in einer von einem männlichen Säugetier bereitgestellten Samenprobe
b) Zugeben von etwas phosphatgepufferter Kochsalzlösung zu der Probe,
c) Abschleudern der Probe auf einen Objektträger,
d) fakultativ Inkubieren des Objektträgers bei 4 °C für 1-1000 Tage,
e) Nachweisen der Expression von CYP24A1 in der Probe auf dem Objektträger, und
f) Bestimmen, dass das männliche Säugetier wahrscheinlich ein normales Fertilitätspotenzial aufweist, wenn das bestimmte Expressionsniveau mindestens gleich einem Vergleichsniveau ist, und/oder Bestimmen, dass das männliche Säugetier wahrscheinlich kein normales Fertilitätspotenzial aufweist, wenn das bestimmte Expressionsniveau unter dem Vergleichsniveau ist.

## Revendications

1. Procédé de prédiction du potentiel de fertilité d'un mammifère mâle, comprenant les étapes suivantes :
a) dans un échantillon de sperme provenant dudit mammifère,
b) déterminer le niveau d'expression de CYP24A1,
c) sélectionner une sensibilité et une sensibilité souhaitées pour un niveau de référence,
d) déterminer que ledit mammifère mâle est susceptible de présenter un potentiel de fertilité normal, si ledit niveau d'expression déterminé est égal ou supérieur au niveau de référence et/ou déterminer que ledit mammifère mâle n'est pas susceptible de présenter un potentiel de fertilité normal, si ledit niveau d'expression déterminé est inférieur au niveau de référence.

2. Procédé selon la revendication 1, dans lequel l'expression de la protéine est détectée à au moins l'un des emplacements dans les cellules de sperme choisi dans le groupe constitué de la région post-acrosomique, le cou, la pièce intermédiaire et l'anneau.

3. Procédé selon la revendication 2, dans lequel l'expression de la protéine est déterminée au niveau de l'anneau.

4. Procédé selon l'une quelconque des revendications 1 à 3, dans lequel ledit niveau d'expression est déterminé par la mesure du niveau d'ARn et/ou de protéine.

5. Procédé selon la revendication 4, dans lequel ladite détermination dudit niveau de protéine est réalisée au moyen d'une méthode choisie dans le groupe consistant en immunobistochimie, immunocytochimie, FACS, ImageStream, test Westem-blot, PCRq, RT-PCR, qRT-PCR, ELISA, Luminex, Multiplex, immunobuvardage, test au TRF, immunochromatographie sur membrane, technique EMIT (Enzyme Multiplied Immunoassay Technique), test RAST, dosage radio-immunologique, immunofluorescence et immunodosage sur bâtonnet sec.

6. Procédé selon la revendication 5, dans lequel ladite détermination est réalisée par immunocytocbimie.

7. Procédé selon la revendication 5, dans lequel ladite détermination est réalisée par la méthode ELISA.

8. Procédé selon l'une quelconque des revendications précédentes, dans lequel le niveau d'expression est combiné au niveau d'expression d'au moins une protéine choisie dans le groupe consistant en Septine 4, Septine 12, Protamine 1, Protamine 2, FGFR1OP1, FGFR1OP2, FGFR1, Calbindine, TRPV6.

9. Utilisation de CYP24A1 en tant que marqueur de potentiel de fertilité d'un échantillon de sperme de mammifère mâle, dans lequel ledit mammifère mâle est susceptible de présenter un potentiel de fertilité normal, si le niveau de CYP24A1 est égal ou supérieur à un niveau de référence et/ou ledit mammifère mâle n'est pas susceptible de présenter un potentiel de fertilité normal, si le niveau de CYP24A1 est inférieur au niveau de référence.

10. Procédé de prédiction du potentiel de fertilité d'un mammifère mâle, comprenant les étapes suivantes :
a) dans un échantillon de sperme provenant d'un mammifère mâle,
b) ajouter un tampon phosphate salin dans l'échantillon,
c) faire ralentir l'échantillon sur une lame,
d) en option, incuber la lame à 4 °C pendant 1 à 1000 jours,
e) détecter l'expression de CYP24A1 dans ledit échantillon sur ladite lame, et
f) déterminer que ledit mammifère mâle est susceptible de présenter un potentiel de fertilité normal si ledit niveau d'expression déterminé est égal ou supérieur à un niveau de référence et/ou déterminer que ledit mammifère mâle n'est pas susceptible de présenter un potentiel de fertilité normal si ledit niveau d'expression déterminé est inférieur au niveau de référence.
